# EUROPEAN PATENT SPECIFICATION

(11) **EP 3 706 759 B1**
(45) Date of publication and mention of the grant of the patent: **15.11.2023**
(21) Application number: 18800124.2
(22) Date of filing: 08.11.2018
(51) Int. Cl.: C12Q 1/689, C12Q 1/6883, A61K 31/7042, A61P 19/00

(54) **HOMOVANILLYL ALCOHOL (HVA), HVA ISOMER, METHODS OF MAKING COMPOSITIONS COMPRISING SUCH COMPOUNDS, AND METHODS USING SUCH COMPOUNDS**
HOMOVANILLYLALKOHOL (HVA), HVA-ISOMER, VERFAHREN ZUR HERSTELLUNG VON ZUSAMMENSETZUNGEN MIT SOLCHEN VERBINDUNGEN UND VERFAHREN ZUR VERWENDUNG SOLCHER VERBINDUNGEN
ALCOOL HOMOVANILLYLIQUE (HVA), ISOMÈRE DE HVA, PROCÉDÉS DE PRÉPARATION DE COMPOSITIONS COMPRENANT DE TELS COMPOSÉS, ET PROCÉDÉS UTILISANT DE TELS COMPOSÉS

(30) Priority: 08.11.2017 EP 17200589; 08.11.2017 EP 17200578; 08.11.2017 EP 17200582; 29.08.2018 US 201862724249 P
(43) Date of publication of application: 16.09.2020
(73) Proprietor: Société des Produits Nestlé S.A., 1800 Vevey (CH)
(72) Inventor: HORCAJADA, Marie Noëlle, 01170 Echenevex (FR); POQUET, Laure, 1077 Servion (CH)
(74) Representative: Kamibayashi, Lynne
(86) International application number: PCT/EP2018/080550
(87) International publication number: WO 2019/092069

(56) References cited:
- WO-A1-2007/118631
- US-A1- 2008 014 322
- US-A1- 2014 256 989
- US-A1- 2016 101 125
- US-A1- 2016 263 139
- US-B2- 9 132 145
- VARELA-EIRIN M ET AL: "Olive-derived oleuropein as a potential treatment for bone and cartilage age-related disorders", OSTEOARTHRITIS AND CARTILAGE, vol. 24, 2016, XP029467430, ISSN: 1063-4584, DOI: 10.1016/J.JOCA.2016.01.730
- KOK-YONG CHIN ET AL: "Olives and Bone: A Green Osteoporosis Prevention Option", INTERNATIONAL JOURNAL OF ENVIRONMENTAL RESEARCH AND PUBLIC HEALTH, vol. 13, no. 8, 26 July 2016 (2016-07-26), page 755, XP055539291, DOI: 10.3390/ijerph13080755
- SANTOS M M ET AL: "Bioconversion of oleuropein to hydroxytyrosol by lactic acid bacteria", WORLD JOURNAL OF MICROBIOLOGY AND BIOTECHNOLOGY, KLUWER ACADEMIC PUBLISHERS, DO, vol. 28, no. 6, 20 March 2012 (2012-03-20) , pages 2435-2440, XP035053735, ISSN: 1573-0972, DOI: 10.1007/S11274-012-1036-Z
- KEITARO HAGIWARA ET AL: "Olive polyphenol hydroxytyrosol prevents bone loss", EUROPEAN JOURNAL OF PHARMACOLOGY, ELSEVIER SCIENCE, NL, vol. 662, no. 1, 12 April 2011 (2011-04-12), pages 78-84, XP028219055, ISSN: 0014-2999, DOI: 10.1016/J.EJPHAR.2011.04.023 [retrieved on 2011-04-27]
- TURNER RUFUS ET AL: "ANTIOXIDANT AND ANTI-ATHEROGENIC ACTIVITIES OF OLIVE OIL PHENOLICS", INTERNATIONAL JOURNAL FOR VITAMIN & NUTRITION RESEARCH, HOGREFE AND HUBER, BERNE, CH, vol. 75, no. 1, 1 January 2005 (2005-01-01), pages 61-70, XP009081671, ISSN: 0300-9831, DOI: 10.1024/0300-9831.75.1.61
- STEFANOS KOUNDOURAS ET AL: "Influence of Vineyard Location and Vine Water Status on Fruit Maturation of Nonirrigated Cv. Agiorgitiko ( Vitis vinifera L.). Effects on Wine Phenolic and Aroma Components", JOURNAL OF AGRICULTURAL AND FOOD CHEMISTRY, vol. 54, no. 14, 1 July 2006 (2006-07-01), pages 5077-5086, XP055541717, US ISSN: 0021-8561, DOI: 10.1021/jf0605446
- DEIANA M ET AL: "Protective effect of simple phenols from extravirgin olive oil against lipid peroxidation in intestinal Caco-2 cells", FOOD AND CHEMICAL TOXICOLOGY, PERGAMON, GB, vol. 48, no. 10, 1 October 2010 (2010-10-01), pages 3008-3016, XP027267298, ISSN: 0278-6915 [retrieved on 2010-08-04]
- RAFAEL DE LA TORRE ET AL: "Protective effect of homovanillyl alcohol on cardiovascular disease and total mortality: virgin olive oil, wine, and catechol-methylathion", THE AMERICAN JOURNAL OF CLINICAL NUTRITION, 26 April 2017 (2017-04-26), XP055542176, US ISSN: 0002-9165, DOI: 10.3945/ajcn.116.145813
- PERES CÁTIA M ET AL: "Novel isolates of lactobacilli from fermented Portuguese olive as potential probiotics", LWT- FOOD SCIENCE AND TECHNOLOGY, vol. 59, no. 1, 10 March 2014 (2014-03-10) , pages 234-246, XP028862673, ISSN: 0023-6438, DOI: 10.1016/J.LWT.2014.03.003
- DEIANA M ET AL: "Protective effect of hydroxytyrosol and its metabolite homovanillic alcohol on H2O2 induced lipid peroxidation in renal tubular epithelial cells", FOOD AND CHEMICAL TOXICOLOGY, PERGAMON, GB, vol. 46, no. 9, 1 September 2008 (2008-09-01), pages 2984-2990, XP024525544, ISSN: 0278-6915, DOI: 10.1016/J.FCT.2008.05.037 [retrieved on 2008-06-07]
- Vincenzo Marsilio ET AL: "Characterisation of an Oleuropein Degrading Strain of Lactobacillus plantarum. Combined E?ects of Compounds Present in Olive Fermenting Brines (Phenols, Glucose and NaCl) on Bacterial Activity", J Sci Food Agric, 1 January 1998 (1998-01-01), pages 520-524, XP055438272, Retrieved from the Internet: URL:http://onlinelibrary.wiley.com/store/1 0.1002/(SICI)1097-0010(199804)76:4<520::AI D-JSFA982>3.0.CO;2-I/asset/982_ftp.pdf?v=1 &t=jc0lqict&s=29896c8f01728cc7c5233f471b47 311b7c9701cd
- AYA UMENO ET AL: "Radical-scavenging Activity and Antioxidative Effects of Olive Leaf Components Oleuropein and Hydroxytyrosol in Comparison with Homovanillic Alcohol", JOURNAL OF OLEO SCIENCE, vol. 64, no. 7, 1 January 2015 (2015-01-01), pages 793-800, XP055582384, JP ISSN: 1345-8957, DOI: 10.5650/jos.ess15042
- ZHONG-JIAN J ET AL: "Phenylpropanoid glycosides from Pedicularis striata pall ssp. Arachnoidea", PHYTOCHEMISTRY, ELSEVIER, AMSTERDAM , NL, vol. 34, no. 4, 1 November 1993 (1993-11-01), pages 1188-1190, XP026632026, ISSN: 0031-9422, DOI: 10.1016/S0031-9422(00)90744-1 [retrieved on 1993-11-01]

## Description

### BACKGROUND

The present disclosure generally relates to oleuropein metabolites and also compositions comprising oleuropein metabolites and further relates to the maintenance or improvement of bone and/or cartilage health. The compositions can additionally or alternatively prevent, alleviate and/or treat bone and/or cartilage disorders. More specifically, the present disclosure relates to homovanillyl alcohol (HVA) and its previously unidentified isomer 3-hydroxy-4-methoxyphenethanol (which can alternatively be named 3-hydroxy-4-methoxyphenethyl alcohol). For example, an oral composition can contain HVA isomer.

Bone mass evolves throughout life and is regulated by genetic, mechanical and hormonal mechanisms. Bone mineral acquisition occurs during childhood, and peak bone mass is achieved around twenty years of age. During this period, bone formation exceeds bone resorption. Later in life, and particularly around the time of the menopause or in the elderly population, bone mass and quality are impaired due to a higher bone turnover, with excessive bone resorption leading to a gradual loss of bone mass, microarchitecture, structure and strength.

Restoration of the balance between bone formation and bone resorption is important to maintain bone. This bone remodelling process is regulated at the cell level of the bone, involving a tight interaction between bone-forming cells (osteoblasts) and bone-resorbing cells (osteoclasts). In a healthy adult (human or animal), the coordinated action of the osteoblasts and osteoclasts maintains bone mass over time and simultaneously ensures remodelling of bone tissue by resorption and *de novo* synthesis of bone.

In a healthy adult, the rate of formation of the osteoclasts and osteoblasts achieves a balance between bone formation and bone resorption. However, in osteoporotic individuals, an imbalance in the process of bone remodelling is produced which culminates in a loss of bone proceeding more rapidly than the rate of formation. This imbalance exists to some extent in most individuals as they age but is accelerated at menopause for women, while it happens later in life for men, and can lead to osteoporosis. Thus, in humans and other mammals, a great variety of disorders is related to unbalanced metabolism of bone resorption and bone formation, leading to osteoporosis.

Moreover, in humans and animals, there are many conditions characterized by the need to increase bone formation. For example, bone fractures necessitate stimulated bone formation in order to accelerate complete repair of the bone. This need is also present in the periodontal diseases, the metastatic diseases of bone, the osteolytic diseases and the conditions under which repair of the connective tissue is required, for example for the cicatrisation or regeneration of defects or traumatisms of cartilage. The stimulation of bone formation or anabolism is also required in the case of primary and secondary hyperparathyroidism, as well as in osteoporosis associated with diabetes and in osteoporosis associated with glucocorticoids.

The current treatments for stimulating bone formation and/or inhibiting bone resorption have limited success.

Another joint disorder is osteoarthritis, which is a first cause of disability in the elderly. Osteoarthritis is a degenerative disease of the articular cartilage of the joint and is the most common form of arthritis. The main features of osteoarthritis are progressive breakdown and loss of the articular cartilage, accompanied by changes to other joint structures such as synovial membrane proliferation, sclerosis and thickness of subchondral bone, osteophyte formation at joint margin, ligament laxity and muscle atrophy, all of which contribute to the clinical symptoms of osteoarthritis. These symptoms include severe pain, stiffness, loss of joint motion and disability.

Currently, no cure exists for osteoarthritis; and therapy is only palliative, aiming at improving symptoms. For example, pain and inflammation are treated using analgesics (such as acetaminophen) and non-steroidal anti-inflammatory drugs (NSAIDs). Furthermore, the use of these drugs is often associated with side effects such as gastrointestinal or cardiovascular risks.

US2016263139A1 relates to anti-inflammatory phytonutrients for use in the treatment or prevention of synovitis.

### SUMMARY

The present invention is defined in the appended claims.

Oleuropein (OE) is the major phenolic compound of the olive tree (*Olea europaea*) and is present at high amounts in unprocessed olive fruits and leaves. The present inventors investigated OE metabolism by identification of its metabolites in urine after ingestion of an olive leaf extract. As set forth in detail later herein, a liquid chromatography-mass spectrometry (LC-MS) method was developed and fully validated for the quantification of hydroxytyrosol, homovanillyl alcohol, and the homovanillyl alcohol isomer 3-hydroxy-4-methoxyphenethanol (alternatively named 3-hydroxy-4-methoxyphenethyl alcohol), which has been identified for the first time.

As also set forth in detail later herein, several metabolites of oleuropein were tested for their effects on chondrocytes. Unexpectedly, homovanillyl alcohol and its isomer in synthetic form are more effective than hydroxytyrosol, which is one of the main circulating forms of oleuropein metabolites.

Further in this regard, the present inventors have provided compositions containing homovanillyl alcohol and/or homovanillyl alcohol isomer or polyphenols extracts enriched with such HVA and/or isomer. Also, as set forth in detail herein, the present inventors have provided nutritional solutions to favour *in-situ* or *in-vivo* production of HVA and/or its isomer to increase efficacy of olive polyphenols.

Further in this regard, the present inventors noted that one possible pathway to obtain and/or enrich a composition in homovanillyl alcohol or its isomer is to subject a source of olive polyphenols to a combination of (a) probiotic and/or enzyme with glycosidase and/or esterase activity and (b) a methyltransferase (e.g., COMT) to obtain the homovanillyl alcohol and/ or isomer. Such reaction may be realized *ex-vivo* (fermentation + methylation) or *in-situ* by co-administration of the source of olive polyphenols with the above combination. Another possible pathway to obtain homovanillyl alcohol or its isomer is to subject a source of olive polyphenol to a probiotic or enzyme with glycosidase and/or esterase activity to produce homovanillyl alcohol and HVA isomer precursors *ex-vivo* (fermentation) or *in-situ* (co-ingestion). Such precursors are then subjected *in-vivo* to mammalian enzyme catechol-O-methyltransferase (COMT) to obtain (e.g., increase the amount of) the homovanillyl alcohol and/or its isomer *in-vivo.* Some of the embodiments disclosed herein advantageously employ at least one of these pathways to achieve surprising and unexpected therapeutic and prophylactic effects.

For example, a preferred embodiment utilizes one or more of the following processes to generate or enrich a source of olive polyphenols with homovanillyl alcohol (or isomer) or favor HVA (and isomer) pathway *in-vivo*:
Probiotics (glycosidase and/or esterase activities) *ex-vivo* (fermentation) or i*n-situ* (co-administration) + COMT (methyl transferase) *in-vivo*
Probiotics (glycosidase and/or esterase activities) *ex-vivo* (fermentation) + methyl transferase *ex-vivo*
Probiotics (glycosidase and/or esterase activities) and methyl transferase (enzyme or probiotic having such activity) (co-administration)
Probiotic having both activities (glycosidase and/or esterase and methyltransferase) (fermentation or co-administration)
Enzyme cocktail (glycosidase and/or esterase activities) + COMT *in vivo*

Enzyme cocktail (glycosidase and/or esterase activities) + methyltransferase enzyme *ex-vivo.*
Enzyme cocktail (glycosidase and/or esterase activities) + bacteria (e.g. probiotic having COMT activity) *ex-vivo*

In such embodiments, at least a portion of the *ex-vivo* methylation can optionally occur first, followed by the esterase/glycosidase activity of the probiotic and/or enzyme; additionally or alternatively, *ex-vivo* methylation can occur after the esterase/glycosidase activity of the probiotic and/or enzyme.

A particularly preferred embodiment utilizes a composition that contains (or is enriched in) HVA or its isomer, either in synthetic form or prepared *ex-vivo* (by fermentation followed by methylation). Another particularly preferred embodiment utilizes a composition that contains a source of olive polyphenols (e.g., olive leaf extract, waste product of olive oil production, an extract containing oleuropein) and also one or more components to produce / favor the HVA pathway during ingestion, for example by co-administration of olive leaf extract + probiotic + COMT or a probiotic having both activities. Yet another particularly preferred embodiment utilizes a composition that contains a source of olive polyphenols + one or more components to produce/ favor HVA precursors (hydroxytyrosol or oleuropein aglycone) during ingestion, the last step of methylation being realized in the body by mammalian methyltransferase.

Accordingly, in an embodiment, the present disclosure provides a method which is not defining the claimed invention, of treating impaired mobility or reducing incidence of impaired mobility in an older adult, the method comprising orally administering a therapeutically or prophylactically effective amount of homovanillyl alcohol and/or homovanillyl alcohol isomer to the older adult. The older adult can be an elderly individual and/or have a condition selected from the group consisting of frailty, pre-frailty, sarcopenia, recovering from sarcopenia, osteoporosis, osteoarthritis, malnutrition, at risk of malnutrition, undergoing rehabilitation, scheduled to undergo rehabilitation within the next year, and combinations thereof.

Additionally or alternatively, a therapeutically or prophylactically effective amount of a precursor of homovanillyl alcohol and/or homovanillyl alcohol isomer can be administered to the older adult, for example precursors of HVA, such as one or more of hydroxytyrosol, oleuropein aglycone, oleuropein, demethyloleuropein, oleuroside, or verbascoside.

In an embodiment, the administration to the older adult is daily for at least one month, for example in a food composition. The food composition can comprise an enriched plant extract that provides at least a portion of the homovanillyl alcohol and/or homovanillyl alcohol isomer, and preferably the enriched plant extract has an increased concentration of homovanillyl alcohol and/or homovanillyl alcohol isomer relative to the starting plant material. The enriched plant extract can be reduced in or substantially free of at least one compound selected from the group consisting of oleuropein, verbascoside, luteolin-7-glucoside, apigenin-7-glucoside, diosmetin-7-glucoside, luteolin, diosmetin, rutin, catechin, tyrosol, hydroxytyrosol, vanillin, vanillic acid and caffeic acid and other phenolic compounds present in the olive leaves extract and waste product of olive oil production (margins); and optionally the enriched plant extract is reduced in or substantially free of at least oleuropein.

Additionally or alternatively, the enriched plant extract can provide at least a portion of the precursor of homovanillyl alcohol and/or homovanillyl alcohol isomer, for example precursors of HVA, such as one or more of hydroxytyrosol, oleuropein aglycone, oleuropein, demethyloleuropein, oleuroside, or verbascoside. In such embodiments, preferably the enriched plant extract has an increased concentration of the precursor relative to the starting plant material. The enriched plant extract can be co-administered with a component selected from the group consisting of one or more bacteria, one or more enzymes, and mixtures thereof, the component having glycosidase activity and esterase activity and optionally also having methyltransferase activity.

In another embodiment, the present disclosure provides a method which is not defining the claimed invention, for stimulating bone formation and/or inhibiting bone resorption in an individual having a condition comprising an imbalance between bone formation and bone resorption, the method comprising orally administering to the individual at least one of (i) an effective amount of a precursor of homovanillyl alcohol and/or homovanillyl alcohol isomer co-administered with a component selected from the group consisting of one or more bacteria, one or more enzymes, and mixtures thereof, the component having glycosidase activity and esterase activity and optionally also having methyltransferase activity, or (ii) an effective amount of homovanillyl alcohol and/or homovanillyl alcohol isomer. The condition can be selected from the group consisting of osteoporosis, Paget's disease, osteolysis adjacent a prosthesis, a metastatic bone disease, hyperthyroidism, hypercalcemia due to a cancer, multiple myelomas, a periodontal disease, osteoarthritis, osteopenia, a bone deficit resulting from a fracture, fracture healing and combinations thereof. The administration to the individual can be daily for at least one month.

In another embodiment, the present disclosure provides a method which is not defining the claimed invention, of treating synovitis in an individual in need thereof, reducing incidence of synovitis in an individual at risk thereof, or treating or reducing incidence of articular cartilage degradation subsequent to synovitis in an individual having or recovering from synovitis, the method comprising orally administering to the individual at least one of (i) a therapeutically or prophylactically effective amount of a precursor of homovanillyl alcohol and/or homovanillyl alcohol isomer co-administered with a component selected from the group consisting of one or more bacteria, one or more enzymes, and mixtures thereof, the component having glycosidase activity and esterase activity and optionally also having methyltransferase activity, or (ii) a therapeutically or prophylactically effective amount of homovanillyl alcohol and/or homovanillyl alcohol isomer. The synovitis can be associated with a condition selected from the group consisting of lupus, gout, rheumatoid arthritis, osteoarthritis, osteochondritis disease, osteoarthrosis and combinations thereof. The administration to the individual can be daily for at least one month.

In another embodiment, the present disclosure provides a method of treating cartilage breakdown or reducing incidence of cartilage breakdown in an individual in need thereof or at risk thereof, the method comprising orally administering to the individual at least one of (i) a therapeutically or prophylactically effective amount of a precursor of homovanillyl alcohol and/or homovanillyl alcohol isomer co-administered with one or more bacteria, having glycosidase activity and esterase activity and optionally also having methyltransferase activity, the bacteria being capable of converting at least a portion of the precursor into homovanillyl alcohol and/or 3-hydroxy-4-methoxyphenethanol or (ii) a therapeutically or prophylactically effective amount of homovanillyl alcohol and/or homovanillyl alcohol isomer. The older adult can be an elderly individual. The administration to the individual can be daily for at least one month.

The references to methods of treatment in paragraphs of this description, e.g. para. 29, are to be interpreted as references to the compounds, pharmaceutical compositions and medicaments of the present invention for use in a method for treatment of the human (or animal) body by therapy, as defined in the appended claims.

In another embodiment, the present disclosure provides a composition which is not defining the claimed invention, at least one of (i) a therapeutically or prophylactically effective amount of a precursor of homovanillyl alcohol and/or homovanillyl alcohol isomer co-administered with a component selected from the group consisting of one or more bacteria, one or more enzymes, and mixtures thereof, the component having glycosidase activity and esterase activity and optionally also having methyltransferase activity, or (ii) a therapeutically or prophylactically effective amount of homovanillyl alcohol and/or homovanillyl alcohol isomer, in an amount effective to achieve an effect selected from the group consisting of (a) maintaining or restoring bone or cartilage metabolism balance, (b) maintaining or improving bone or cartilage health, (c) maintaining or improving mobility in an older adult, (d) treating or preventing synovitis, and (e) combinations thereof. The composition is a food product comprising a component selected from the group consisting of protein, carbohydrate, fat and combinations thereof.

In another embodiment, the present disclosure provides a method which is not defining the claimed invention, of making a composition for achieving an effect selected from the group consisting of (a) maintaining or restoring bone or cartilage metabolism balance, (b) maintaining or improving bone or cartilage health, (c) maintaining or improving mobility in an older adult, (d) treating or preventing synovitis, and (e) combinations thereof. The method comprises: forming an enriched plant extract comprising at least one of (i) homovanillyl alcohol and/or homovanillyl alcohol isomer or (ii) a precursor of homovanillyl alcohol and/or homovanillyl alcohol isomer, the enriched plant extract is formed from a starting plant material comprising olive polyphenols, the forming of the enriched plant extract comprises an *ex-vivo* treatment of the starting plant material with an effective amount of a bacteria and/or enzyme having activities that perform a step in bioconversion of at least a portion of the olive polyphenols in the starting plant material into the at least one of (i) homovanillyl alcohol and/or homovanillyl alcohol isomer or (ii) a precursor of homovanillyl alcohol and/or homovanillyl alcohol isomer; and including the enriched plant extract in the composition.

In an embodiment, the composition comprises at least one ingredient selected from the group consisting of protein, carbohydrate, and fat in addition to the enriched plant extract.

In an embodiment, the starting plant material comprises one or more other compounds in addition to homovanillyl alcohol and/or homovanillyl alcohol isomer, and the enriched plant material has a ratio of homovanillyl alcohol and/or homovanillyl alcohol isomer relative to the one or more other compounds that is higher than the ratio in the starting plant material.

In an embodiment, the starting plant material comprises one or more other compounds in addition to precursor of homovanillyl alcohol and/or homovanillyl alcohol isomer, and the enriched plant material has a ratio of the precursor relative to the one or more other compounds that is higher than the ratio in the starting plant material.

In an embodiment, the starting plant material comprises olive leaf extract or waste products of olive oil production.

In an embodiment, the activity of the bacteria and/or enzyme comprises at least one of beta-glucosidase activity or esterase activity and optionally further comprises methyltransferase activity.

In an embodiment, the precursor comprises at least one of oleuropein or a derivative thereof.

In an embodiment, the method further comprises quantifying the amount of homovanillyl alcohol and/or homovanillyl alcohol isomer in the enriched plant extract and/or the composition.

In an embodiment, the bacteria and/or enzyme comprises a bacteria in an amount sufficient to form *ex vivo* an amount of an intermediate compound that is converted *in vivo* into a therapeutically or prophylactically effective amount of homovanillyl alcohol and/or homovanillyl alcohol isomer by a methyltransferase in an individual to whom the composition is administered.

In an embodiment, the bacteria and/or enzyme comprises a bacteria and an enzyme in a total amount sufficient to form *ex vivo* a therapeutically or prophylactically effective amount of homovanillyl alcohol and/or homovanillyl alcohol isomer in the enriched plant extract.

In an embodiment, the bacteria comprises a first bacterial strain having a first enzymatic activity and a second bacterial strain having a second enzymatic activity different than the first enzymatic activity in a total amount sufficient to form *ex vivo* a therapeutically or prophylactically effective amount of homovanillyl alcohol and/or homovanillyl alcohol isomer in the enriched plant extract.

In an embodiment, the enzyme comprises a first enzyme having a first enzymatic activity and a second enzyme having a second enzymatic activity different than the first enzymatic activity in a total amount sufficient to form *ex vivo* a therapeutically or prophylactically effective amount of homovanillyl alcohol and/or homovanillyl alcohol isomer in the enriched plant extract.

In another embodiment, the present disclosure provides a method which is not defining the claimed invention, of making a composition for achieving an effect selected from the group consisting of (i) maintaining or restoring bone or cartilage metabolism balance, (ii) maintaining or improving bone or cartilage health, (iii) maintaining or improving mobility in an older adult, (iv) treating or preventing synovitis, and (v) combinations thereof. The method comprises: adding a precursor of homovanillyl alcohol and/or homovanillyl alcohol isomer to at least one ingredient selected from the group consisting of protein, carbohydrate, and fat; and adding to the at least one ingredient an amount of an enzyme capable of converting *in vivo* at least a portion of the precursor into an effective amount of (a) homovanillyl alcohol and/or homovanillyl alcohol isomer and/or (b) an intermediate compound that can be subjected to methyltransferase in an individual to whom the composition is administered to form homovanillyl alcohol and/or homovanillyl alcohol isomer.

In an embodiment, the precursor is provided by olive leaf extract added to the at least one ingredient.

In an embodiment, the precursor comprises at least one of oleuropein or a derivative thereof.

In an embodiment, the method further comprises quantifying the amount of homovanillyl alcohol and/or homovanillyl alcohol isomer in the composition.

In another embodiment, the present disclosure provides a method which is not defining the claimed invention, of selecting at least one probiotic strain, the method comprising: (a) identifying a probiotic strain having a β-glucosidase encoding gene by *in silico* analysis and/or β-glucosidase activity by *in vitro* analysis of a plurality of strains; (b) identifying a probiotic strain having an esterase encoding gene by *in silico* analysis and/or esterase activity by *in vitro* analysis of a plurality of strains; (c) fermenting a starting amount of oleuropein and/or hydroxytyrosol with i) a bacterial culture of the probiotic strain identified in step (a) as having β-glucosidase activity and the probiotic strain identified in step (b) as having esterase activity or ii) a bacterial culture of the probiotic strain identified in steps (a) and (b) as having both β-glucosidase and esterase activity; (d) identifying whether the enzymatic or fermentation in step (c) forms homovanillyl alcohol and/or homovanillyl alcohol isomer; (e) measuring a proportion of the starting amount of oleuropein and/or hydroxytyrosol that is remaining as a function of fermentation time in step (c); and (f) identifying a probiotic strain that consumes at least 10 wt.% of the starting amount of oleuropein and/or hydroxytyrosol in step (c) over a time period of less than twenty-four hours and that forms homovanillyl alcohol and/or homovanillyl alcohol isomer in step (c).

In an embodiment, identification of a probiotic strain in step (a) by *in silico* analysis is performed by providing the genome of the probiotic strain and performing a sequence similarity search for a gene coding for a β-glucosidase enzyme in the genome of the probiotic strain; and identification of a probiotic strain in step (b) by *in silico* analysis is performed by providing the genome of the probiotic strain and performing a sequence similarity search for a gene coding for a esterase enzyme in the genome of the probiotic strain.

In an embodiment, the method further comprises incorporating the probiotic strain identified in step (f) into a food product. The incorporating of the probiotic strain identified in step (f) into the food product can comprise adding the probiotic strain identified in step (f) to at least one edible ingredient selected from the group consisting of a protein, a carbohydrate, and a lipid.

In another embodiment, the present disclosure provides a method which is not defining the claimed invention, of treating or preventing cartilage breakdown, the method comprising administering to an individual in need thereof or at risk thereof at least one of (i) a therapeutically or prophylactically effective amount of homovanillyl alcohol and/or homovanillyl alcohol isomer or (ii) a combination of a precursor of homovanillyl alcohol and/or homovanillyl alcohol isomer and a probiotic capable of converting at least a portion of the precursor into homovanillyl alcohol and/or homovanillyl alcohol isomer, the combination is administered in an amount that provides a therapeutically or prophylactically effective amount of homovanillyl alcohol and/or homovanillyl alcohol isomer. The references to methods of treatment in paragraphs of this description, e.g. para. 50, are to be interpreted as references to the compounds, pharmaceutical compositions and medicaments of the present invention for use in a method for treatment of the human (or animal) body by therapy.

In another embodiment, the present disclosure provides an oral composition which is not defining the claimed invention, comprising homovanillyl alcohol isomer. In yet another embodiment, the present disclosure provides an oral composition comprising: a precursor of homovanillyl alcohol and/or homovanillyl alcohol isomer; and a component selected from the group consisting of one or more bacteria, one or more enzymes, and a mixture thereof, the component having at least one activity selected from the group consisting of glycosidase activity, esterase activity, and methyltransferase activity, preferably at least methyltransferase activity. These oral compositions can optionally further comprise one or more olive polyphenols.

An advantage of one or more embodiments provided by the present disclosure is mobility in aging, frail or pre-frail individuals, for example such individuals who are recovering from rehabilitation or at risk of sarcopenia, and particularly bone and joint benefits in such individuals.

Another advantage of one or more embodiments provided by the present disclosure is to treat or reduce incidence of osteopenia (mild loss of bone mass), promote bone growth in young individuals, and/or treat or reduce incidence of disorders linked with an unbalanced ratio between bone formation and bone resorption.

Yet another advantage of one or more embodiments provided by the present disclosure is to stimulate bone formation or inhibit bone resorption in a subject suffering from osteoporosis, osteolysis adjacent a prosthesis, periodontal disease, osteoarthritis and/or osteopenia.

Still another advantage of one or more embodiments provided by the present disclosure is to enhance bone formation and/or cartilage anabolism; treat or reduce incidence of cartilage breakdown; and/or limit synovitis and the subsequent articular cartilage degradation (osteoarthritis) during aging.

Additional features and advantages are described in, and will be apparent from, the following Detailed Description and the Figures.

### BRIEF DESCRIPTION OF DRAWINGS

**FIG. 1** shows the chemical structure of oleuropein.
**FIG. 2** shows the proposed metabolism pathway of oleuropein by mammalian and microbial enzymes, based on the findings reported in the literature.
**FIGS. 3A-3C** are graphs showing identification of oleuropein aglycone glucuronide of oleuropein aglycone glucuronide in Example 1 disclosed herein; **FIGS. 4A** and 4C are urine sample not treated with glucuronidase and sulfatase, and **FIGS. 4B** and **4D** are urine sample treated with glucuronidase and sulfatase.
**FIGS. 4A-4C** are graphs showing CID product ion spectrum of [M-H]⁻ species at *m*/*z* 553 in Example 1 disclosed herein.
**FIGS. 5** show the product ion spectrum of [M-H]⁻ at *m*/*z* 377 (RT=9.56) and identity of the fragments in Example 1 disclosed herein, based on the literature (the major fragments are bold in the table 2).
**FIG. 6A** shows the chemical structure of oleuropein aglycone; and **FIG. 6B** shows its aldehydic form.
**FIGS. 7A-7C** show identification of oleuropein aglycone sulfate in Example 1 disclosed herein; **FIG. 7A** is urine sample not treated with glucuronidase and sulfatase, **FIG. 7B** is urine sample treated with glucuronidase and sulfatase, and **FIG. 7C** is product ion spectrum of [M-H]⁻ at *m*/*z* 457 (RT=9.52).
**FIGS. 8A-8D** show product ion spectrum of [M-H]⁻ with *m*/*z* at 329 in Example 1 disclosed herein.
**FIGS. 9A-9D** show product ion spectrum of [M-H]⁻ with *m*/*z* at 233 in Example 1 disclosed herein.
**FIGS. 10A** and **10B** show MS³ spectrum (409 → 233) in Example 1 disclosed herein.
**FIG. 11** shows the search for HVOH glucuronide by MRM with the transition 343 → 113 in Example 1 disclosed herein.
**FIGS. 12A-12C** show MS³ analysis with first precursor [M-H]⁻ at *m*/*z* 167 and second precursor ion at *m*/*z* 152 in Example 1 disclosed herein.
**FIG. 13A** shows the chemical structure of homovanillyl alcohol; and **FIG. 13B** shows its isomer (3-hydroxy-4-methoxyphenethanol or 3-hydroxy-4-methoxyphenethyl alcohol).
**FIG. 14** shows the oxidation of hydroxytyrosol (HT) and homovanillyl alcohol (HVOH).
**FIG. 15** shows estimation of the quantity of each potential oleuropein metabolite in urine at basal (left column for each subject, blue) and after intake of olive leaf extract (right column for each subject, orange) in Example 1 disclosed herein; the vertical axis is area under the peak, and the horizontal axis is subjects 1 to 5.
**FIG. 16A** shows collagen expression data from Example 2 disclosed herein.
**FIGS. 16B** and **16C** show matrix metalloproteinase expression data from Example 2 disclosed herein.
**FIGS. 17A** and **17B** show inflammation-related gene expression data from Example 2 disclosed herein.
**FIGS. 18A** and **18B** show collagen anabolism and catabolism data from Example 2 disclosed herein.

### DETAILED DESCRIPTION

### Definitions

Some definitions are provided hereafter. Nevertheless, definitions may be located in the "Embodiments" section below, and the above header "Definitions" does not mean that such disclosures in the "Embodiments" section are not definitions.

All percentages expressed herein are by weight of the total weight of the composition unless expressed otherwise. As used herein, "about," "approximately" and "substantially" are understood to refer to numbers in a range of numerals, for example the range of -10% to +10% of the referenced number, preferably -5% to +5% of the referenced number, more preferably -1% to +1% of the referenced number, most preferably -0.1% to +0.1% of the referenced number.

All numerical ranges herein should be understood to include all integers, whole or fractions, within the range. Moreover, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9, and so forth.

As used in this disclosure and the appended claims, the singular forms "a," "an" and "the" include plural referents unless the context clearly dictates otherwise. Thus, for example, reference to "a component" or "the component" includes two or more components.

The words "comprise," "comprises" and "comprising" are to be interpreted inclusively rather than exclusively. Likewise, the terms "include," "including" and "or" should all be construed to be inclusive, unless such a construction is clearly prohibited from the context. Nevertheless, the compositions disclosed herein may lack any element that is not specifically disclosed herein. Thus, a disclosure of an embodiment using the term "comprising" includes a disclosure of embodiments "consisting essentially of" and "consisting of" the components identified. A composition "consisting essentially of" contains at least 50 wt.% of the referenced components, preferably at least 75 wt.% of the referenced components, more preferably at least 85 wt.% of the referenced components, most preferably at least 95 wt.% of the referenced components. Any embodiment disclosed herein can be combined with any other embodiment disclosed herein.

The term "and/or" used in the context of "X and/or Y" should be interpreted as "X," or "Y," or "X and Y." Similarly, "at least one of X or Y" should be interpreted as "X," or "Y," or "X and Y." For example, "at least one of homovanillyl alcohol or 3-hydroxy-4-methoxyphenethanol" should be interpreted as "homovanillyl alcohol" or "3-hydroxy-4-methoxyphenethanol" or "both homovanillyl alcohol and 3-hydroxy-4-methoxyphenethanol."

Where used herein, the terms "example" and "such as," particularly when followed by a listing of terms, are merely exemplary and illustrative and should not be deemed to be exclusive or comprehensive. As used herein, a condition "associated with" or "linked with" another condition means the conditions occur concurrently, preferably means that the conditions are caused by the same underlying condition, and most preferably means that one of the identified conditions is caused by the other identified condition.

The terms "food," "food product" and "food composition" mean a product or composition that is intended for ingestion by an individual such as a human and provides at least one nutrient to the individual. As used herein, a "food product" encompasses beverages. A food product typically includes at least one of a protein, a lipid, a carbohydrate and optionally includes one or more vitamins and minerals. The compositions of the present disclosure, including the many embodiments described herein, can comprise, consist of, or consist essentially of the elements disclosed herein, as well as any additional or optional ingredients, components, or elements described herein or otherwise useful in a diet.

Probiotics are micro-organisms that when administered in adequate amounts confer health benefits to the host.

A "subject" or "individual" is a mammal, such as livestock or a companion animal (e.g., dog or cat), preferably a human. "Prevention" includes reduction of risk, incidence and/or severity of a condition or disorder. As used herein, an "effective amount" is an amount that treats or prevents a deficiency, treats or prevents a disease or medical condition in an individual, or, more generally, reduces symptoms, manages progression of the disease, or provides a nutritional, physiological, or medical benefit to the individual.

The term "elderly" in the context of a human means an age from birth of at least 60 years, preferably above 63 years, more preferably above 65 years, and most preferably above 70 years. The term "older adult" in the context of a human means an age from birth of at least 45 years, preferably above 50 years, more preferably above 55 years, and includes elderly individuals.

"Sarcopenia" is defined as the age-associated loss of muscle mass and functionality (including muscle strength and gait speed).

As used herein, "frailty" is defined as a clinically recognizable state of increased vulnerability resulting from aging-associated decline in reserve and function across multiple physiologic systems such that the ability to cope with everyday or acute stressors is compromised. In the absence of an established quantitative standard, frailty has been operationally defined by Fried et al (Journal of Gerontology: MEDICAL SCIENCES 2001, Vol. 56A, No. 3, M146-M156) as meeting three out of five phenotypic criteria indicating compromised energetics: (1) weakness (grip strength in the lowest 20% of population at baseline, adjusted for gender and body mass index), (2) poor endurance and energy (self-reported exhaustion associated with VO2 max), (3) slowness (lowest 20% of population at baseline, based on time to walk 15 feet, adjusting for gender and standing height), (4) low physical activity (weighted score of kilocalories expended per week at baseline, lowest quintile of physical activity identified for each gender; e.g., less than 383 kcal/week for males and less than 270 kcal/week for females), and/or unintentional weight loss (10 lbs. in past year). Fried LP, Tangen CM, Walston J, et al., "Frailty in older adults: evidence for a phenotype." J. Gerontol. A. Biol. Sci. Med. Sci. 56(3):M146-M156 (2001). A pre-frail stage, in which one or two of these criteria are present, identifies a high risk of progressing to frailty.

As used herein, an "effective amount" is an amount that prevents a deficiency, treats a disease or medical condition in an individual or, more generally, reduces symptoms, manages progression of the diseases or provides a nutritional, physiological, or medical benefit to the individual. The relative terms "improved," "increased," "enhanced" and the like refer to the effects of the composition disclosed herein, namely a composition comprising at least one of homovanillyl alcohol or 3-hydroxy-4-methoxyphenethanol, relative to a composition lacking these compounds but otherwise identical.

The terms "treatment" and "treat" include both prophylactic or preventive treatment (that prevent and/or slow the development of a targeted pathologic condition or disorder) and curative, therapeutic or disease-modifying treatment, including therapeutic measures that cure, slow down, lessen symptoms of, and/or halt progression of a diagnosed pathologic condition or disorder; and treatment of patients at risk of contracting a disease or suspected to have contracted a disease, as well as patients who are ill or have been diagnosed as suffering from a disease or medical condition. The terms "treatment" and "treat" do not necessarily imply that a subject is treated until total recovery. The terms "treatment" and "treat" also refer to the maintenance and/or promotion of health in an individual not suffering from a disease but who may be susceptible to the development of an unhealthy condition. The terms "treatment" and "treat" are also intended to include the potentiation or otherwise enhancement of one or more primary prophylactic or therapeutic measures. As non-limiting examples, a treatment can be performed by a patient, a caregiver, a doctor, a nurse, or another healthcare professional.

The references to methods of treatment in paragraphs of this description, e.g. para. 91, are to be interpreted as references to the compounds, pharmaceutical compositions and medicaments of the present invention for use in a method for treatment of the human (or animal) body by therapy.

As used herein, "associated with" and "linked with" mean occurring concurrently, preferably means caused by the same underlying condition, and most preferably means that one of the identified conditions is caused by the other identified condition.

The term "unit dosage form", as used herein, refers to physically discrete units suitable as unitary dosages for human and animal subjects, each unit containing a predetermined quantity of the composition disclosed herein in an amount sufficient to produce the desired effect, in association with a pharmaceutically acceptable diluent, carrier or vehicle. The specifications for the unit dosage form depend on the particular compounds employed, the effect to be achieved, and the pharmacodynamics associated with each compound in the host.

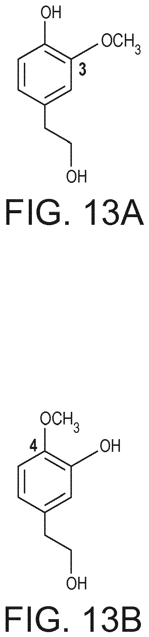

"Homovanillyl alcohol" ("HVA" or "HVOH") is the chemical compound shown in **FIG.** 13A. "Homovanillyl alcohol isomer," alternatively referenced as "isoHVA," "isoHVOH," "3-hydroxy-4-methoxyphenethanol" or "3-hydroxy-4-methoxyphenethyl alcohol," is the chemical compound shown in **FIG. 13B****.**

### Embodiments

An aspect of the present disclosure is a composition comprising a homovanillyl alcohol and/or homovanillyl alcohol isomer. In an embodiment, at least a portion of the homovanillyl alcohol and/or homovanillyl alcohol isomer can be obtained by chemical synthesis, for example by subjecting of hydroxytyrosol to methylation of the catechol moiety (on 3-OH or 4-OH of the phenyl ring), e.g., by catechol-*O*-methyl-transferase (COMT) on either hydroxyl group of hydroxytyrosol.

Additionally or alternatively, at least a portion of the homovanillyl alcohol and/or homovanillyl alcohol isomer can be obtained by extraction. For example, at least a portion of the homovanillyl alcohol and/or homovanillyl alcohol isomer can be obtained by extraction from a plant such as a plant in the *Oleaceae* family, preferably one or more of the stems, the leaves, the fruits or the stones of a plant belonging to the *Oleaceae* family such as *Olea europaea* (olive tree).

In an embodiment, the composition comprises a plant extract (e.g., from a plant in the *Oleaceae* family) that provides at least a portion of the homovanillyl alcohol and/or homovanillyl alcohol isomer. In such embodiments, the plant extract is preferably enriched in homovanillyl alcohol and/or homovanillyl alcohol isomer such that the plant extract has a ratio of homovanillyl alcohol and/or homovanillyl alcohol isomer relative to one or more other components of the starting plant material that is higher than the ratio in the starting plant material. In a particular embodiment, the composition does not contain any exogenous homovanillyl alcohol and/or homovanillyl alcohol isomer, i.e., the enriched plant extract provides all of the homovanillyl alcohol and/or homovanillyl alcohol isomer in the composition.

As a non-limiting example, the starting plant material can be subjected to enzymatic treatment that can comprise bioconversion of at least a portion of the oleuropein therein to homovanillyl alcohol and/or homovanillyl alcohol isomer. The resultant extract can be used in the composition to provide at least a portion of the homovanillyl alcohol and/or homovanillyl alcohol isomer therein. In a particular embodiment, one or more of the enzymes has at least one of beta-glucosidase activity or esterase activity, preferably both. In some embodiments, one or more of the enzymes has methyltransferase activity. The enzyme can be identified by quantitatively assaying the capability to convert the precursor to homovanillyl alcohol and/or homovanillyl alcohol isomer or an intermediate compound.

In an embodiment, at least a portion of the homovanillyl alcohol and/or homovanillyl alcohol isomer is isolated such that the extract (and/or the composition as a whole) is substantially free or completely free from one or more other components of the starting plant material from which the homovanillyl alcohol and/or homovanillyl alcohol isomer is isolated. An extract "substantially free" of a compound means the extract has less than 1.0 wt.% of the compound, preferably less than 0.5 wt.% of the compound, more preferably less than 0.2 wt.%, most preferably less than 0.1 wt.%. Similarly, a composition "substantially free" of a compound means the composition has less than 1.0 wt.% of the compound, preferably less than 0.5 wt.% of the compound, more preferably less than 0.2 wt.%, most preferably less than 0.1 wt.%.

Non-limiting examples of compounds in plant material from *Oleaceae* (other than homovanillyl alcohol and/or homovanillyl alcohol isomer) include oleuropeosides (oleuropein and verbascoside); flavones (luteolin-7-glucoside, apigenin-7-glucoside, diosmetin-7-glucoside, luteolin and diosmetin); flavonols (rutin); flavan-3-ols (catechin) and substituted phenols (tyrosol, hydroxytyrosol, vanillin, vanillic acid and caffeic acid). Further regarding these compounds, an embodiment of the composition comprises a plant extract from *Oleaceae* that is enriched in homovanillyl alcohol and/or homovanillyl alcohol isomer such that the plant extract has a ratio of homovanillyl alcohol and/or homovanillyl alcohol isomer relative to one or more of these compounds that is higher relative to the ratio in the starting plant material subjected to the extraction. Additionally or alternatively, the extract (and/or the composition as a whole) can be reduced in, substantially free, or completely free of one or more of these compounds. "Reduced" in one or more of these compounds means that the concentration of the compound is less in the extract than in the starting material.

Another aspect of the present disclosure is a composition comprising a precursor of homovanillyl alcohol and/or homovanillyl alcohol isomer, for example at least one of oleuropein or hydroxytyrosol, and a probiotic that performs at least one step in bioconversion of the precursor into homovanillyl alcohol and/or homovanillyl alcohol isomer. The probiotic can be identified from a plurality of probiotic strains by quantitatively assaying the capability to convert the precursor to homovanillyl alcohol and/or homovanillyl alcohol isomer, for example by one or more of: identifying the probiotic strain using genomic *in silico* analysis on a plurality of strains, assessing the *in vitro* bioconversion of the precursor by the probiotic strain (microbial deglycosylation and/or hydrolysis), determining if the probiotic strain improves the *in vivo* formation of homovanillyl alcohol and/or homovanillyl alcohol isomer (optionally determining the effect of the carrier matrix), assessing *in vitro* efficacy using primary chondrocyte and osteoblast cultures, and/or using a clinical trial investigating the effect of the precursor and the probiotic strain on bone and joint health outcomes.

In a particular embodiment, the probiotic strain (or mixture of probiotic strains) is identified by a method comprising:
(a) identifying a probiotic strain having a β-glucosidase encoding gene by *in silico* analysis and/or β-glucosidase activity by *in vitro* analysis of a plurality of strains;
(b) identifying a probiotic strain having an esterase encoding gene by *in silico* analysis and/or esterase activity by *in vitro* analysis of a plurality of strains;
(c) fermenting the precursor (e.g., oleuropein or hydroxytyrosol) with (i) a bacterial culture of a probiotic strain identified in step (a) as having β-glucosidase activity and a probiotic strain identified in step (b) as having esterase activity or (ii) a bacterial culture of a probiotic strain identified in steps (a) and (b) as having both β-glucosidase and esterase activity;
(d) identifying whether the metabolites formed during the fermentation of step (c) includes homovanillyl alcohol and/or homovanillyl alcohol isomer;
(e) measuring the proportion of oleuropein remaining as a function of fermentation time in step (c); and
(f) selecting a probiotic strain or probiotic strain mixture wherein the probiotic strain or probiotic strain mixture consumes at least 10.0 wt.% of oleuropein in step (c) over a time period of less than 24, and wherein the metabolites formed during the fermentation of step (c) comprise homovanillyl alcohol and/or homovanillyl alcohol isomer.

In some embodiments, the probiotic comprises a first probiotic that forms an intermediate metabolite from the precursor and a second probiotic that forms homovanillyl alcohol and/or homovanillyl alcohol isomer from the intermediate.

In an embodiment, the precursor and the probiotic can be co-administered, for example administered sequentially in separate compositions. The term "sequentially" means that the precursor and the probiotic are administered in a successive manner such that the precursor is administered at a first time without the probiotic, and the probiotic is administered at a second time subsequent to the first time without the precursor. The time between sequential administrations may be, for example, one or several seconds, minutes or hours in the same day; one or several days or weeks in the same month; or one or several months in the same year. The order of sequential administration may be reversed.

Further in this regard, "co-administration" of the precursor and the probiotic and/or enzyme means that the probiotic is administered to an individual who has consumed the precursor and/or will consume the precursor and does not necessarily mean that they are administered at the same time in the same composition. Although concurrent administration in the same composition is indeed preferred, the present disclosure is not limited to this embodiment. For example, "co-administration" also encompasses administration within the same day, and preferably within the same hour. If the precursor and the probiotic are comprised within the same composition, the precursor may be isolated from the probiotic to prevent fermentation of the precursor on storage. For example, the probiotic may be encapsulated separately from the precursor. Preferably the probiotic only reacts with the precursor after administration, for example in the digestive tract of the individual.

Some individuals can have bacterial flora containing probiotics that will already have activity converting the precursor to homovanillyl alcohol and/or homovanillyl alcohol isomer. Therefore, in some cases administration of the composition enhances the therapeutic and/or prophylactic effect of these probiotics by providing more of the bacteria.

Another aspect of the present disclosure is a method in which a therapeutically or prophylactically effective amount of one or more of the compositions disclosed herein is administered to an individual. For example, one or more doses of the composition (e.g., one or more unit dosage forms) can be administered to provide a therapeutically or prophylactically effective amount of homovanillyl alcohol and/or homovanillyl alcohol isomer to the individual.

Non-limiting examples of such methods include a method of maintaining or restoring bone metabolism balance by stimulating bone formation and/or preventing bone resorption; a method of treating or preventing a disorder linked to cartilage turnover by stimulating cartilage anabolism through inhibiting or decreasing cartilage breakdown; a method of preventing or treating a bone disorder (e.g., a disorder associated with an unbalanced bone formation: bone resorption ratio, such as osteoporosis) or for maintaining bone health; a method of stimulating bone formation and/or cartilage anabolism during a growth period of a young individual; stimulating bone formation and/or cartilage anabolism in adults in order to increase maximal bone mass (e.g., in perimenopausal women, healthy adults, healthy aging adults, and adults who are pre-osteoarthritic); a method of treating or preventing bone loss which occurs with aging (osteopenia); a method of treating a bone deficiency resulting from a fracture; and a method of treating or preventing synovitis (e.g., synovitis associated with lupus, gout, or arthritis such as one or more of rheumatoid arthritis, osteoarthritis, osteochondritis disease, and osteoarthrosis).

In some embodiments, the composition is administered to treat or prevent impaired mobility in an older adult, for example by maintaining or improving joint functionality (e.g., bone functionality and/or cartilage functionality). The older adult can have a condition selected from the group consisting of frailty, pre-frailty, sarcopenia, recovering from sarcopenia, osteoporosis, osteoarthritis, malnutrition, at risk of malnutrition, undergoing rehabilitation, scheduled to undergo rehabilitation (e.g., within the next year, preferably within the next six months, more preferably within the next month), and combinations thereof.

The individual to whom the composition is administered can be at risk of the disorder or condition, in which case the effective amount of the composition is a prophylactically effective dose; or the individual can have the disorder or condition, in which case the effective amount of the composition is a therapeutically effective dose. In some embodiments, the methods comprise identifying the individual as having the condition or being at risk of the condition before the administration.

In an embodiment, the composition is administered to the individual for a time period of at least one month; preferably at least two months, more preferably at least three, four, five or six months; most preferably for at least one year. During the time period, the composition can be administered to the individual at least one day per week; preferably at least two days per week, more preferably at least three, four, five or six days per week; most preferably seven days per week. The composition can be administered in a single dose per day or in multiple separate doses per day.

In some embodiments, the composition is used in one of the methods disclosed by U.S. Patent App. Publ. Nos. 2016/0045519, 2016/0120891 and 2016/0263139.

The effective amount of the composition varies with the particular composition, the age and condition of the recipient, and the particular disorder or disease being treated. Nevertheless, in a general embodiment, the composition can be administered to the individual in an amount that provides 0.01 mg to 2 g of the homovanillyl alcohol and/or homovanillyl alcohol isomer per day, preferably from 0.1 mg to 1 g of the homovanillyl alcohol and/or homovanillyl alcohol isomer per day, and more preferably from 1 mg to 200 mg of the homovanillyl alcohol and/or homovanillyl alcohol isomer per day.

Another aspect of the present disclosure is a method of making a composition for achieving an effect selected from the group consisting of (i) maintaining or restoring bone or cartilage metabolism balance, (ii) maintaining or improving bone or cartilage health, (iii) maintaining or improving mobility in an older adult, (iv) treating or preventing synovitis, and (v) combinations thereof. The compositions and methods can additionally or alternatively prevent, alleviate and/or treat bone and/or cartilage disorders. The composition is preferably a food product.

In an embodiment, the method comprises adding homovanillyl alcohol and/or homovanillyl alcohol isomer to an ingredient selected from the group consisting of a protein, a carbohydrate, a lipid, and mixtures thereof. Additionally or alternatively, the method can comprise adding, to an ingredient selected from the group consisting of a protein, a carbohydrate, a lipid, and mixtures thereof, a combination of a precursor of homovanillyl alcohol and/or homovanillyl alcohol isomer (e.g., oleuropein and hydroxytyrosol) and a probiotic capable of at least one step of bioconversion of the precursor.

In an embodiment, the method comprises subjecting one or more ingredients of the composition (e.g., an extract of *Oleaceae*) with an enzyme that provides enrichment of the one or more ingredients in homovanillyl alcohol and/or homovanillyl alcohol isomer. This enzymatic treatment can be performed before and/or after the one or more ingredients are added to the other ingredients of the composition.

The composition (e.g., food product) can be made prior to administration (e.g., the composition is made, packaged, and then purchased by a consumer who administers the composition to themselves or to another individual) or can be made substantially simultaneous to administration (the composition is made less than 30 minutes before administration, preferably less than 15 minutes before administration, more preferably less than 10 minutes before administration, most preferably less than 5 minutes before administration, by an individual who administers the composition to themselves or to another individual).

The composition can comprise an effective amount of the homovanillyl alcohol and/or homovanillyl alcohol isomer. Additionally or alternatively, the composition can comprise an effective amount of a combination of the precursor and the probiotic. For example, a single serving or dose of the composition can comprise the effective amount, and a package can contain one or more of the servings or doses.

The composition can comprise a food additive selected from the group consisting of acidulants, thickeners, buffers or agents for pH adjustment, chelating agents, colorants, emulsifiers, excipients, flavor agents, minerals, osmotic agents, a pharmaceutically acceptable carrier, preservatives, stabilizers, sugars, sweeteners, texturizers, vitamins, minerals and combinations thereof.

The composition can comprise an additional ingredient for bone quality, for example protein, vitamin C, vitamin D, vitamin K2, calcium, phosphorus, magnesium, zinc, hesperidin (flavanone), or combinations thereof. The composition can comprise an additional for joint quality comprising at least one ingredient for short-term joint quality, for example, glucosamine (e.g., glucosamine sulfate), chondroitin (e.g., chondroitin sulfate), hyaluronic acid (e.g., a rooster comb extract rich in hyaluronic acid) or combinations thereof (preferably at least hyaluronic acid), and/or at least one ingredient for long-term joint quality, for example vitamin C, another polyphenol (e.g., curcumin, quercetin and/or rutin), omega-3 fatty acids, or combinations thereof. Non-limiting examples of other suitable additional ingredients for joint quality include collagen, hydrolyzed collagen, *Boswellia serrata,* rose hip, and combinations thereof.

The protein can be whey, e.g., native whey, intact unhydrolyzed whey, whey protein concentrate, whey protein isolate, acid whey, sweet whey, modified sweet whey (sweet whey from which the caseino-glycomacropeptide has been removed), a fraction of whey protein, or whey protein hydrolysate; casein; a vegetable protein such as soy protein; and combinations thereof. The casein may be provided in free form or in the form of a salt, for example, a sodium salt, a calcium salt or a potassium salt. Although the protein can comprise vegetable protein, in some embodiments the composition is gluten-free.

The protein may be extensively hydrolyzed protein hydrolysates prepared from acid or enzyme treated animal and vegetable proteins, such as casein hydrolysate, whey hydrolysate, casein/whey hydrolysate, soy hydrolysate, and mixtures thereof. "Extensively hydrolyzed" protein hydrolysates means that the intact protein is hydrolyzed into peptide fragments in which a majority of the peptide fragments have a molecular weight less than 1,000 Daltons, preferably at least about 75% and most preferably at least about 95% of the peptide fragments having a molecular weight less than about 1,000 Daltons. Free amino acids and synthetic short peptide chains may be substituted for or added to the protein hydrolysates.

In an embodiment, the protein comprises whey protein micelles as described in U.S. Patent App. Pub. No. 2009/0035437 and its counterpart EP1839492A1 and as further characterized in C. Schmitt et al., Soft Matter 6:4876-4884 (2010) where they are referred to as whey protein microgels (WPM). Particularly, whey protein micelles are the micelles comprised in the whey protein micelles concentrate obtained by the process as disclosed in U.S. Patent App. Pub. No. 2009/0035437 and its counterpart EP1839492A1. Therein, the process for the production of whey protein micelles concentrate comprises the steps of a) adjusting the pH of a whey protein aqueous solution to a value between 3.0 and 8.0; b) subjecting the aqueous solution to a temperature between 80 and 98 °C; and c) concentrating the dispersion obtained in step b). Thereby, the micelles produced have an extremely sharp size distribution, such that more than 80% of the micelles produced have a size smaller than 1 micron in diameter and preferably are between 100 nm and 900 nm in size. The whey protein micelles can be in liquid concentrate or in powder form. Importantly, the basic micelle structure of the whey proteins is conserved, whether in the liquid concentrate form, the powder form, or reconstituted from the powder, for example in water. The whey protein micelles are physically stable in dispersion, as a powder as well as during spray-drying or freeze-drying.

Non-limiting examples of suitable carbohydrates include starch, sucrose, lactose, glucose, fructose, corn syrup solids, maltodextrin, modified starch, amylose starch, tapioca starch, corn starch, xylitol, sorbitol or combinations thereof. Non-limiting examples of suitable lipids include vegetable fat (such as olive oil, corn oil, sunflower oil, high-oleic sunflower, rapeseed oil, canola oil, hazelnut oil, soy oil, palm oil, coconut oil, blackcurrant seed oil, borage oil, lecithins, and the like), animal fats (such as milk fat), or combinations thereof. The source of fat may also be less refined versions of these fats (e.g., olive oil for polyphenol content).

The composition can be in any oral nutritional form, e.g. as a health drink, as a ready-made drink, optionally as a soft drink, including juices, milk-shake, yogurt drink, smoothie or soy-based drink; in a food bar; or dispersed in foods of any sort, such as baked products, cereal bars, dairy bars, snack-foods, soups, breakfast cereals, muesli, candies, tabs, cookies, biscuits, crackers (such as rice crackers), and dairy products.

The composition may be in the form of tablets, capsules, pastilles or a liquid, for example. The composition may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins or the like), adsorbents, carriers, fillers, co-compounds, dispersing agents, wetting agents, processing aids (solvents), flowing agents, taste masking agents, weighting agents, jellifying agents and gel forming agents.

### EXAMPLES

The following non-limiting examples present experimental data supporting the compounds, compositions, and methods disclosed herein.

### Example 1: A validated LC-MS method for the quantification in urine of the oleuropein metabolites hydroxytyrosol, homovanillyl alcohol, and the newly identified isomer of homovanillyl alcohol

### Overview

Oleuropein (OE) is the major phenolic compound of the olive tree (*Olea europaea*) and is present at high amounts in unprocessed olive fruits and leaves. Its metabolism was investigated by the identification of its metabolites in urine after ingestion of an olive leaf extract. An LC-MS method was developed and fully validated for the quantification of hydroxytyrosol, homovanillyl alcohol and the homovanillyl alcohol isomer 3-hydroxy-4-methoxyphenethanol (alternatively named 3-hydroxy-4-methoxyphenethyl alcohol), which has been identified for the first time.

Specifically, urine samples were enzymatically treated to hydrolyze the glucuronide and sulfate moieties and release the free forms which were quantified. After sample purification by liquid-liquid extraction, the chromatographic separation was operated on an Acquity^{™} UPLC^{®} BEH C18 column (1.7 µm, 2.1 × 150 mm). The mobile phase consisted of 1 mmol/L ammonium acetate buffer and methanol under gradient conditions at a flow-rate of 0.25 mL/min. Hydroxytyrosol, homovanillyl alcohol, and the homovanillyl alcohol isomer 3-hydroxy-4-methoxyphenethanol (alternatively named 3-hydroxy-4-methoxyphenethyl alcohol) were identified and quantified by high performance liquid chromatography-electrospray ionization tandem mass spectrometry (HPLC-ESI-MS/MS) and by comparison with their corresponding chemically synthesized standards.

The results were as follows. The main oleuropein metabolites excreted in urine after olive leaf extract intake were identified as hydroxytyrosol, homovanillyl alcohol, and homovanillyl alcohol isomer. This is the first time that homovanillyl alcohol isomer is reported as an oleuropein metabolite. These compounds were also detected as conjugates with glucuronide or sulfate moieties, or both as for hydroxytyrosol. Several oleuropein algycones, also found conjugated with sulfate or glucuronide have been detected. Neither the parent compound oleuropein nor the metabolite elenolic acid could be detected under free or conjugated forms.

The method performance was assessed by the determination of the linearity, limit of detection, limit of quantification, precision, trueness and uncertainty.

In conclusion, an LC-MS method has been developed and validated for the simultaneous quantification of hydroxytyrosol, homovanillyl alcohol and homovanillyl alcohol isomer.

### Introduction

An olive leaf extract was tested for its potential efficacy on the improvement of joint functionality and the limitation of cartilage breakdown in over-55 healthy consumers with mild to moderate knee discomfort and mobility concerns.

Oleuropein (OE) is the major phenolic compound of olive tree (*Olea europaea* L.), representing about 40 % of the olive leaf extract used in this study, and OE is hypothesized to be the main active compound of this extract. In parallel to the measurement of health benefits from intake of the olive leaf extract, the absorption of oleuropein was followed to support the benefit data with proof of bioavailability of the active compound. To achieve this analysis, an analytical method was required.

The ideal situation to follow the absorption of a compound such as oleuropein is to measure the plasma circulating concentrations of the ingested compound and its metabolites, at different time points following the intake. However, it was not technically feasible to collect blood samples at different time points following ingestion of the olive leaf extract and to draw such kinetics for all the subjects involved in the study. An alternative could have been to collect only one blood sample after intake, with the risk to choose the wrong time point and to miss the peak of absorption, knowing the inter-individual variability of absorption. For these reasons, OE absorption was followed by the measurement of the parent compound and/or its metabolites excreted in pooled urine samples collected during an overnight period of time.

The circulating concentration and kinetics of absorption of phenolic compounds and their metabolites in biological fluids (plasma or urine) depends on the matrix in which the compounds are ingested, the dose ingested, the time point of sample collection, the microflora of each individual, and the metabolism of each individual. To develop the most appropriate analytical approach for the measurement of OE absorption, a pilot study was conducted. Specifically, five subjects collected urine at baseline (before intake of the olive leaf extract) and between the intakes of two capsules of the olive leaf extract.

This pilot study allowed:
(1) to test for the technical feasibility of samples collection, e.g., bottles, storage and stability.
(2) identification of urine OE metabolites after intake of olive leaf extract and selection of the most representative OE metabolites.
(3) development of the analytical method, e.g., sample preparation, detection, and chromatographic separation.
(4) determination of the concentrations ranges to target during method validation.

### Materials and Methods

The analytical method (sample preparation, deconjugation conditions, chromatographic detection and separation) was first developed by spiking chemical standards which were commercially available in commercial urine. The deconjugation conditions were determined by using the conjugates available, hydroxytyrosol sulfate and homovanillyl alcohol glucuronide. The commercially available chemical standards were 3,4-Dihydroxybenzoic acid; 3,4-Dihydroxyphenylacetic acid; Homovanillic acid; Homovanillyl alcohol; Homovanillyl alcohol glucuronide (vanillyl methanol glucuronide); Hydroxytyrosol; Hydroxytyrosol sulfate (hydroxytyrosol sulfate triethylammonium salt); and Vanillic acid.

Briefly, the sample preparation consisted of a liquid-liquid extraction with ethyl acetate after a protein precipitation using acidified methanol.

Regarding chromatographic analysis, Oleuropein metabolites were separated by reversed-phase ultra-performance liquid chromatography using a C18-column Acquity UPLC BEH C18 1.7 µm, 2.1 mm×150 mm, (Waters AG, Switzerland). The system consisted of a LC system Acquity^{™} UPLC^{®} (Waters AG, Switzerland) coupled with a Qtrap 5500 Mass spectrometer detector equipped with an electrospray ionization source (ABSciex, Switzerland). Detection was performed in the negative operating mode at unit resolution.

The analyses of oleuropein metabolites conjugates (sulfates and glucuronides) required the use of acid in the mobiles phases. Solvent A consisted of 0.1% formic acid in water, while methanol was the solvent B. However, the detection of the unconjugated forms was better with 0.1 % acetic acid in water as solvent A for hydroxytyrosol and 1 mmol/L ammonium acetate buffer at pH 5.1 as solvent A for homovanillyl alcohol.

### Results

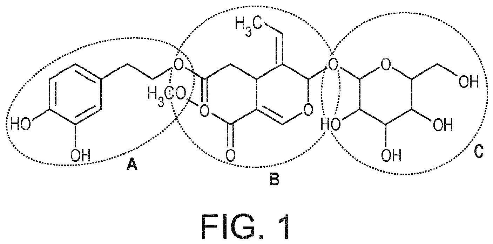

The approach for the detection of oleuropein metabolites in urine was based on the literature review of OE bioavailability. As shown in **FIG. 1****,** OE structure can be detailed into three units corresponding to hydroxytyrosol (A), elenolic acid (B) and glucose (C).

Due to its size, OE cannot cross the intestinal barrier by passive paracellular diffusion through the tight junctions. Due to its polarity and the presence of the sugar moiety, OE cannot cross the barrier by transcellular diffusion and would require an active transport which has not been reported in the literature so far. The deglycosylation of the molecule before absorption, as demonstrated for other polyphenols, seems to be a prerequisite to its permeation. Considered as a xenobiotic by the body, once absorbed, most probably as an aglycone (de-glycosylated), OE is conjugated by mammalian cells (intestinal and hepatic, mainly) with a sulfate, a glucuronide, or a methyl group to render it more polar and clear it through urine. Indeed, glucuronides, sulfates and methyl conjugates of OE aglycone have been reported in the literature (Garcia-Villalba et al. Eur J Nutr (2014) 53:1015-1027).

Because of its low absorption in the upper part of the intestine, OE reaches the colon where it is degraded by the microflora. This microbial metabolism leads to the production of oleuropein aglycone as well as hydroxytyrosol, elenolic acid and glucose. The phenolic compounds produced can then be absorbed and conjugated, or not, with sulfate, glucuronide or methyl group. Before their absorption, they can also be further metabolized by the microflora into smaller compounds by oxidation reaction, metabolites which can be themselves conjugated with sulfate, glucuronide or methyl group after absorption.

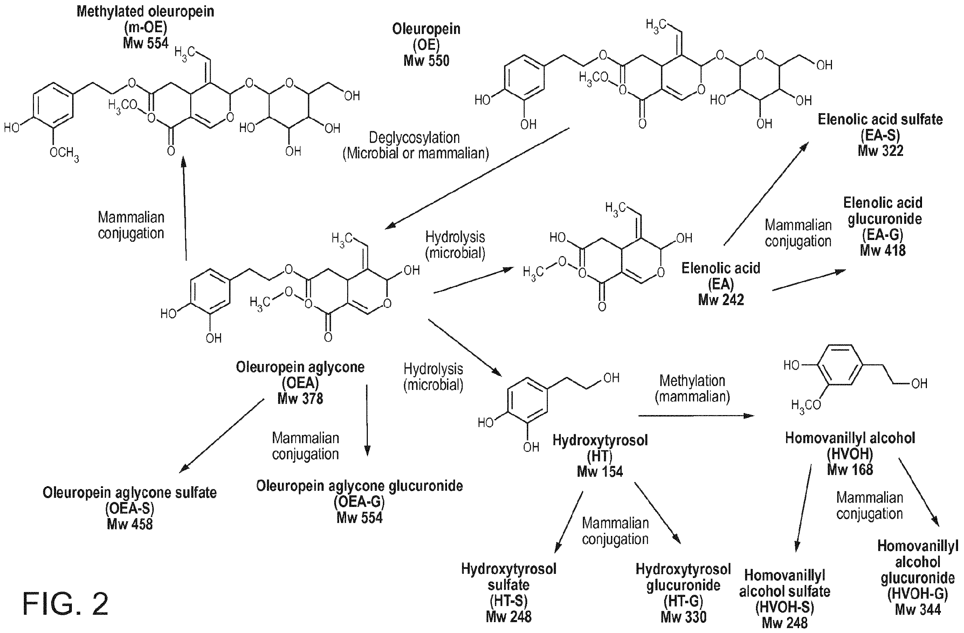

**FIG. 2** shows a proposed metabolism pathway of oleuropein by mammalian and microbial enzymes, based on the findings reported in the literature. Detection and identification of potential oleuropein metabolites in urine samples was based on this proposed metabolism pathway.

Specifically, a preliminary analysis of the urine samples collected after intake of the olive leaf extract was performed by electrospray ionization (ESI) in the negative ion mode, scanning for the different [M-H]⁻ ions at the theoretical *m*/*z* of the expected oleuropein metabolites and conjugates (**Table 1**).

**Table 1 is listing theoretical m/z for [M-H]⁻ ions of potential oleuropein metabolites.**

| **Abbreviation** | **Compound** | ***Theoretical m*/*z* of corresponding [M-H]⁻** |
|---|---|---|
| OE | Oleuropein | 539 |
| OEA-G | Oleuropein aglycone glucuronide | 553 |
| OEA-S | Oleuropein aglycone sulfate | 457 |
| OEA | Oleuropein aglycone | 377 |
| EA-G | Elenolic acid glucuronide | 417 |
| EA-S | Elenolic acid sulfate | 321 |
| EA | Elenolic acid | 241 |
| HT-G | Hydroxytyrosol glucuronide | 329 |
| HT-S | Hydroxytyrosol sulfate | 233 |
| HT | Hydroxytyrosol | 153 |
| HVOH-G | Homovanillyl alcohol glucuronide | 343 |
| HVOH-S | Homovanillyl alcohol sulfate | 247 |
| HVOH | Homovanillyl alcohol | 167 |

Once ions were detected, their identity was confirmed by:
- disappearance of the potential glucuronide or sulfate forms after use of deconjugating enzyme (treatment of the sample with the combination of a sulfatase and a glucuronidase)
- specific presence after intake of olive leaf extract compared with urine samples collected before intake of olive leaf extract
- increase of concentration of the corresponding unconjugated parent after enzymatic treatment
- MS² or MS³ fragmentation and identification of the detected compounds
- comparison of the product ion scan with the one obtained for the corresponding chemical standard (if commercially available)

Oleuropein (OE), as parent compound, was not detected in urine, either because not present or because present in quantities under the limit of detection.

Neither ion of elenolic acid sulfate nor of elenolic acid glucuronide (EA-S or EA-G) could be detected. Several peaks were detected when searching for [M-H]⁻ ions at *m*/*z* 241 but none of them could be confirmed as being EA.

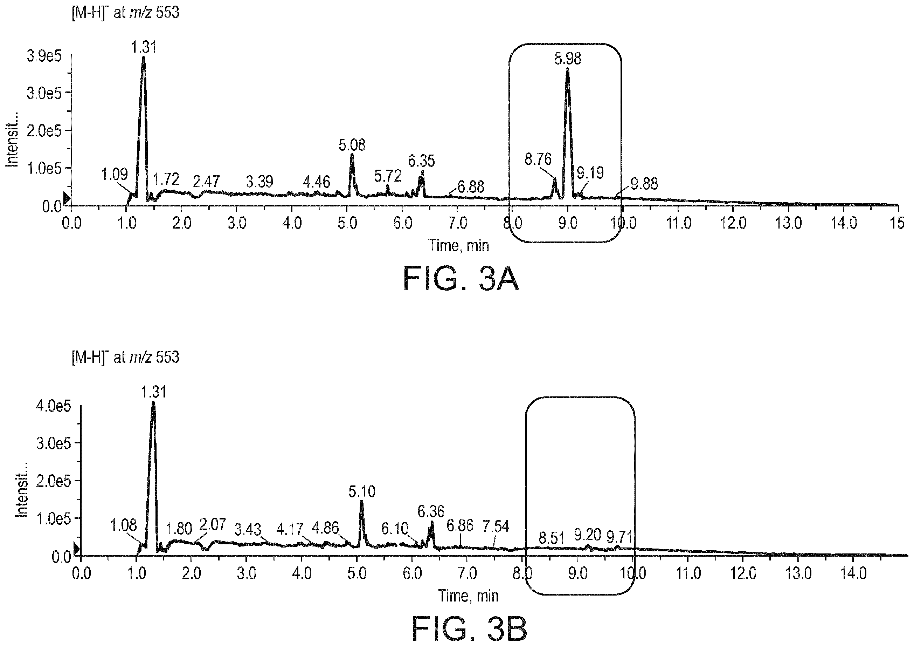

Searching for oleuropein aglycone glucuronide (OEA-G), several [M-H]⁻ ions at *m*/*z* 533 were detected, as shown in **FIG. 3A****.** When the samples were treated with deconjugating enzymes (glucuronidase and sulfatase), two of these peaks (RT=8.76 and 8.98 min) disappeared (**FIG. 3B**).

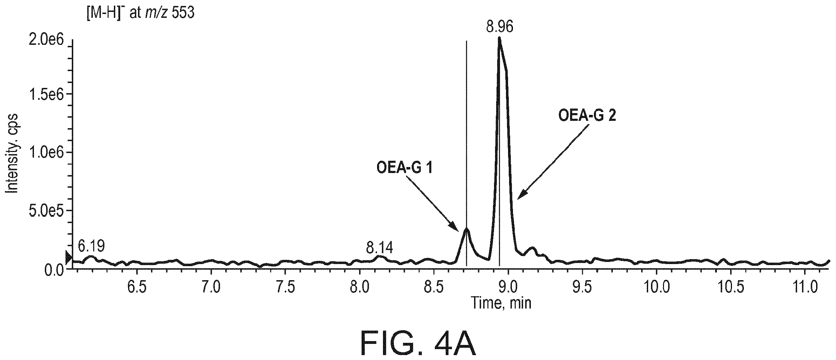

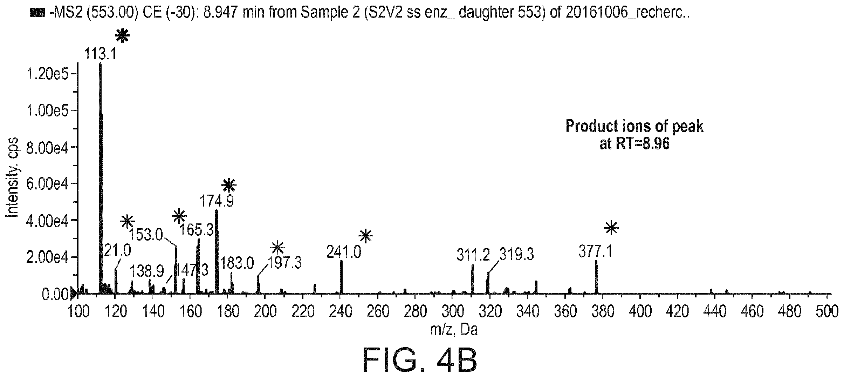

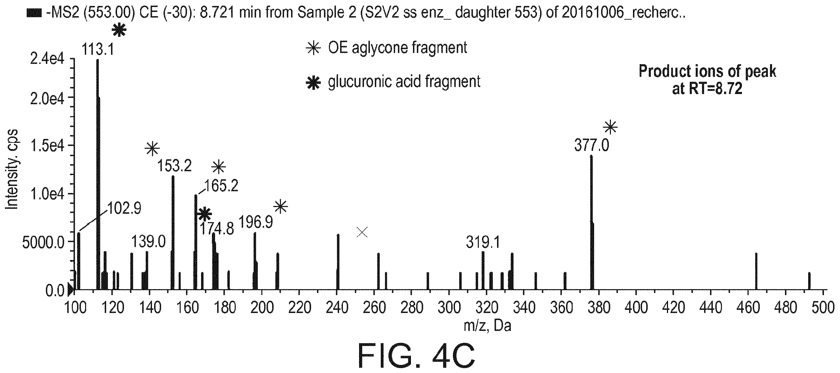

At the same time, two peaks appeared by searching for [M-H]⁻ ions at *m*/*z* 377 (OEA). MS² analysis confirmed that the peaks at RT=8.76 and 8.98 min (**FIGS. 4A-4C**) are OEA glucuronides. Indeed, the CID product ion spectrum of the [M-H]⁻ species at *m*/*z* 553 showed, for both peaks, fragments at:
- *m*/*z* at 377: neutral loss of 176 amu corresponding to dehydrated glucuronic acid, due to the cleavage of glycosidic bond from OEA-G, with charge retention by the aglycone moiety (OEA)
- *m*/*z* at 241: [M-H]⁻ ion of elenolic acid
- *m*/*z* at 153: [M-H]⁻ ion of hydroxytyrosol
- *m*/*z* at 165: reported as elenolic acid fragment (Dierkes et al. 2012)
- *m*/*z* at 197: [OEA-H-C9H8O4]- (Zhong et al. 2012).

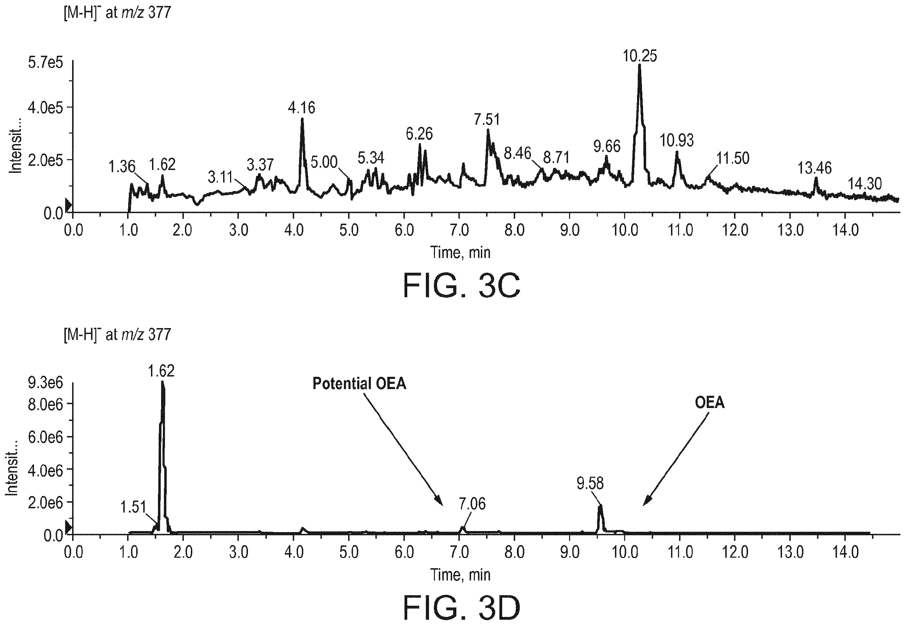

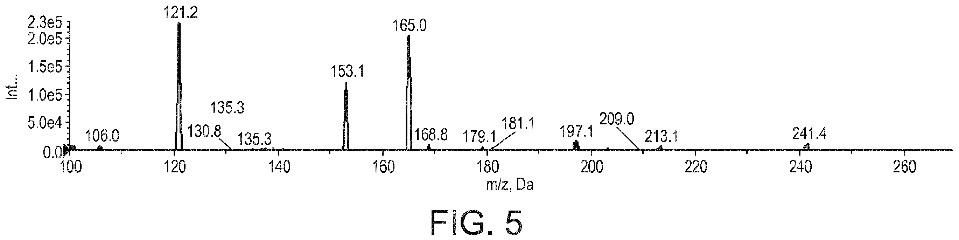

Searching for OEA by scanning for *m*/*z* at 377, two [M-H]⁻ ions were detected and corresponding chromatogram peaks increased in sized or appeared (depending on the subjects) when samples collected after intake of the olive leaf extract were treated with deconjugating enzymes (**FIG. 3D**), compared with the same samples before deconjugation (**FIG. 3C**). The identity of OEA could be confirmed by MS² for the peak at RT=9.56 min. **FIGS. 5** and **Table 2** show the CID product ion spectrum of [M-H]⁻ ion at *m*/*z* 377 with RT=9.56 min, which exhibited fragment ions at *m*/*z* reported in the literature for OEA. These data suggest that the peak at RT=9.56 min is oleuropein aglycone. The peak at RT=7.06 min was too small to obtain intense enough product ion spectrum and to identify the compound with certainty.

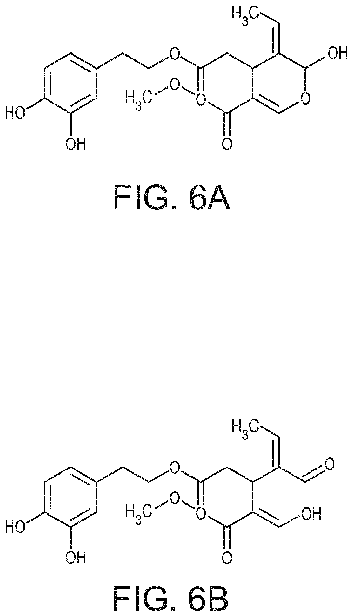

Both peaks obtained for the ions at *m*/*z* 377 (at RT=9.56 min confirmed as OEA, and RT=7.06 min, not fully confirmed as OEA) were also present in basal sample of 3 subjects over the 5 involved in the pilot study. Oleuropein aglycone could come from the consumption of olive oil or olives by these subjects (Di Maio et al. Food Chemistry 138 (2013) 1381-1391). Indeed, during maturation or processing of olives to prepare oil, OE is hydrolyzed and OEA is produced (Fu et al. Rapid Commun. Mass Spectrom. 2009; 23: 51-59). Together with its decarboxymethylated form (3,4-DHPEA-EDA), OEA, also named 3,4-dihydroxyphenylethanol-elenolic acid (3,4-DHPEA), is one of the most abundant compounds of olive oil. The two anions at *m*/*z* 377 could correspond to oleuropein aglycone (**FIG. 6A**) and its aldehydic form (**FIG. 6B**), which has been reported to exist as different stereoisomers (Pérez-Trujillo et al. 2010). However, further analyses would be required to confirm the identity of the peak at RT=7.06 min.

**Table 2: show the product ion spectrum of [M-H]⁻ at m/z 377 (RT=9.56) and identity of the fragments in Example 1 disclosed herein, based on the literature (the major fragments are bold). Dierkes et al. J. Agric. Food Chem. 2012, 60, 7597-7606; Kendall et al. (2012) Food Chemistry 130: 651-659; Zhong et al. Chromatographia (2012) 75:111-129**

| **Fragment ions *m*/*z*** | **Fragment ion identity** | **Reference** |
|---|---|---|
| 241.4 | EA ion | (Kendall et al. 2012) |
| 197.1 | OEA fragment ion ([M-H-C₉H₈O₄]⁻) | (Zhong et al. 2012) |
| 181.1 | OEA fragment ion | (Dierkes et al. 2012) |
| **165.0** | EA fragment ion | (Dierkes et al. 2012) |
| **153.1** | Hydroxytyrosol ion | (Kendall et al. 2012, Zhong et al. 2012) |
| **121.2** | OEA fragment ion | (Dierkes et al. 2012) |

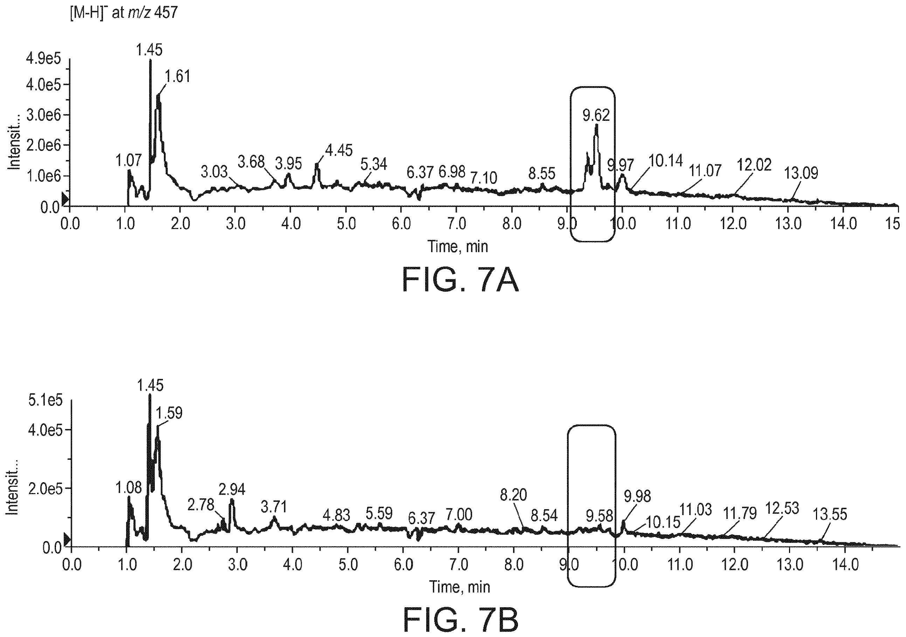

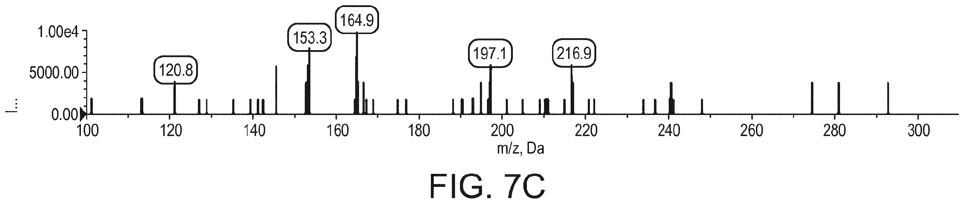

Searching for potential oleuropein sulfate (OE-S), two ions at *m*/*z* 457 were detected (**FIG. 7A**), two close peaks which disappeared when samples were enzymatically treated (**FIG**. **7B**). The CID product ion spectrum of [M-H]⁻ at *m*/*z* 457 (**FIG. 7C**) exhibited fragment ions reported in the literature as being specific of OEA: *m*/*z* at 120.8, *m*/*z* at 153.3, *m*/*z* at 164.9 and *m*/*z* at 197.1 (as detailed above). The fragment ion at *m*/*z* 216.9 has been reported by Fu et *al.* (Fu et al. 2009) for different isomers of olive oil OEA.

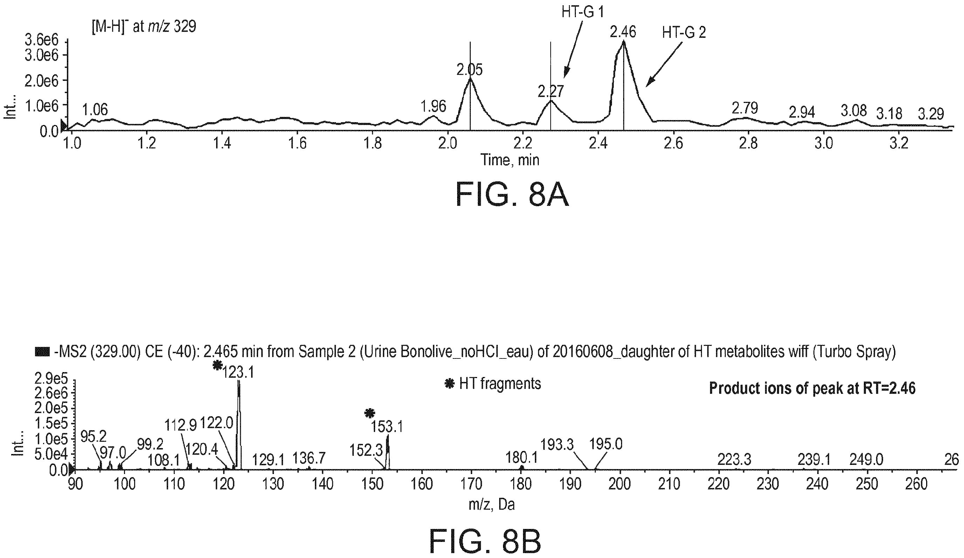

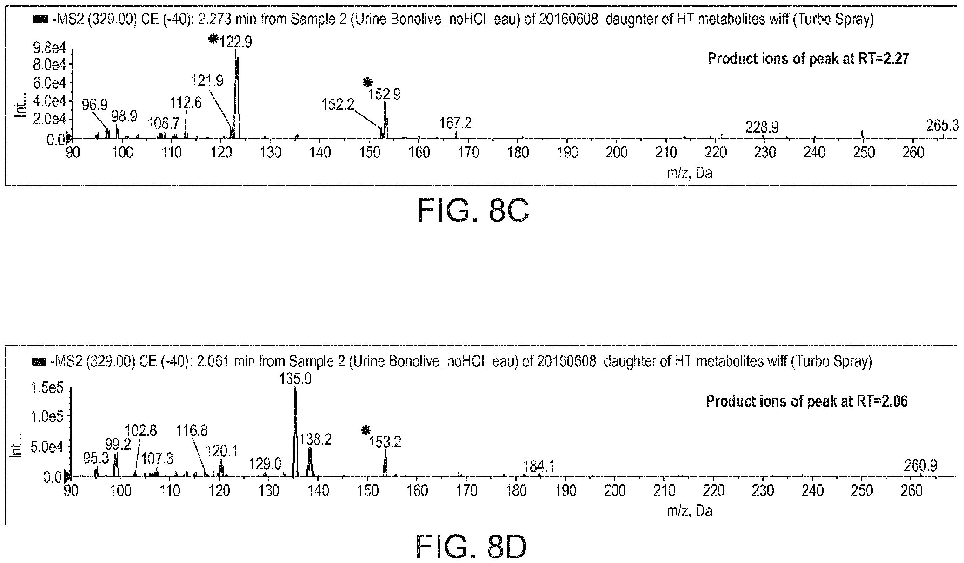

Searching for hydroxytyrosol glucuronide (HT-G), three ions at *m*/*z* 329 (RT=2.05, 2.27 and 2.46 min; **FIGS. 8A-8D**) were detected. They disappeared after enzymatic treatment, suggesting that they correspond to conjugated compounds. The CID product ion spectrum of ions at *m*/*z* 329 for RT=2.27 and 2.46 min exhibited fragments at *m*/*z* 153 (hydroxytyrosol ion) and 123 (due to a loss of 30 amu from 153, corresponding to a loss of CH₂OH from hydroxytyrosol). The peak at RT=2.05 min gave product ions at *m*/*z* 153 but also at *m*/*z* 135 and 138.2, fragment ions which could not be identified as resulting from HT. These findings strongly suggest that at least two forms of HT glucuronide exist (RT=2.27 and 2.46 min). These two compounds could correspond to the isomers of conjugated hydroxytyrosol with the glucuronide attached either on the 3-OH or on the 4-OH. More work would be needed to clarify the identity of the compound at RT=2.05 min.

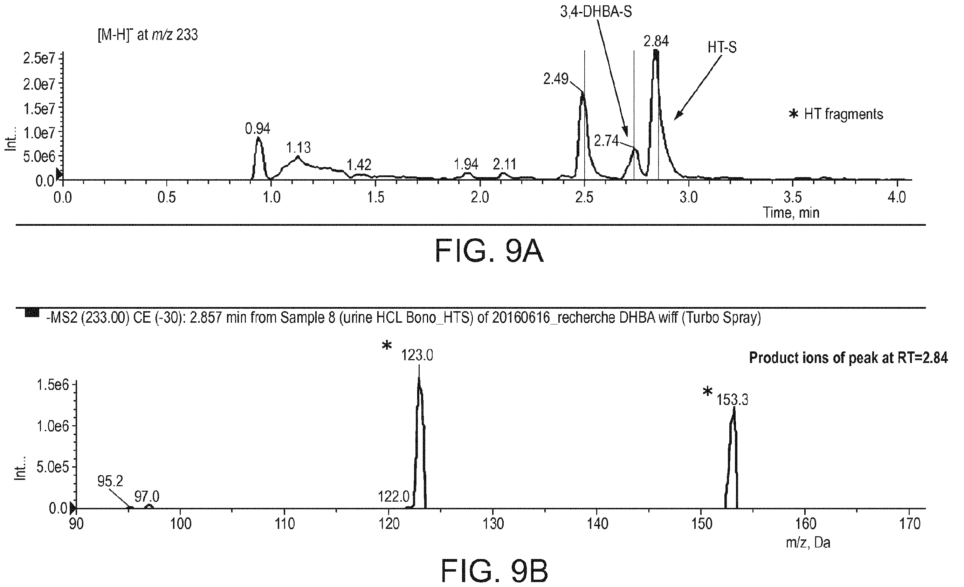

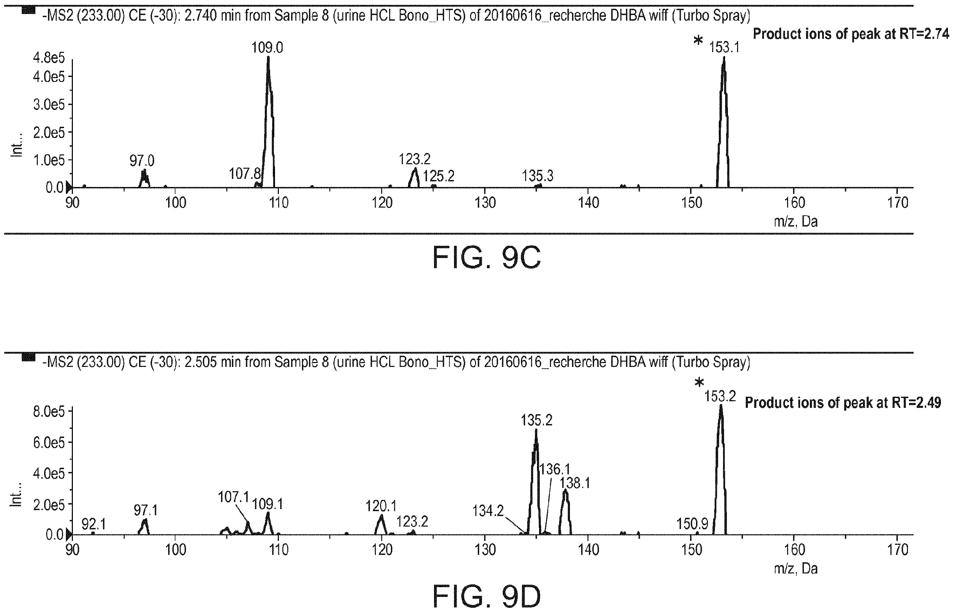

Searching for hydroxytyrosol sulfate (HT-S), three ions at *m*/*z* 233 (**FIGS. 9A-9D**) were also detected, which disappeared with enzymatic treatment. CID product ion spectrum of [M-H]⁻ species at *m*/*z* 233 showed that only the peak at RT=2.84 min exhibited fragments representative of HT (*m*/*z* at 153 and 123). The ion detected at RT=2.74 min gave fragments ions at *m*/*z* 153 but also 109. HT appears to have the same mass as 3,4-dihydroxybenzoic acid (3,4-DHBA), and the second fragment of the product ion spectrum of 3,4-DHBA has an *m*/*z* at 109. These results suggest that the peak at RT=2.74 min is 3,4-dihydroxybenzoic acid sulfate (3,4-DHBA-S). The third ion found at *m*/*z* 233 (RT=2.50 min) gave product ion fragments at *m*/*z* 153 and the same unidentified fragments as for the product ion analysis of [M-H]⁻ at *m*/*z* 329, meaning *m*/*z* 135 and 138.2. This must correspond to the same compound but sulfated. More investigation would be required to identify this compound giving fragment ions at *m*/*z* 135 and 138.2.

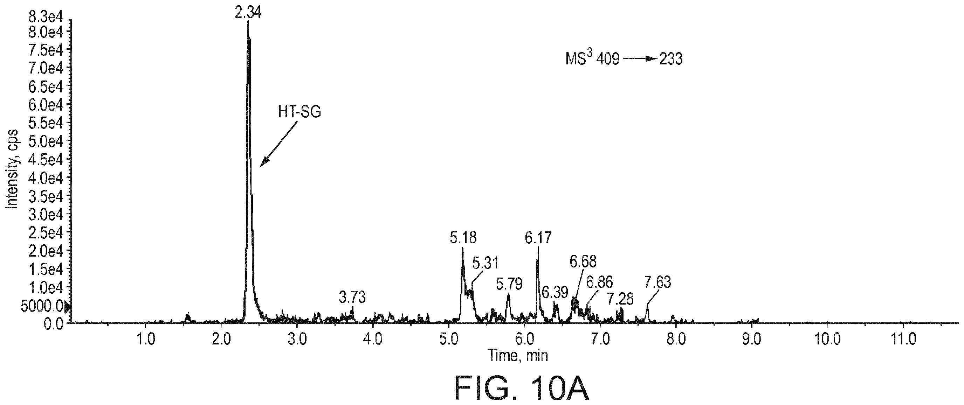

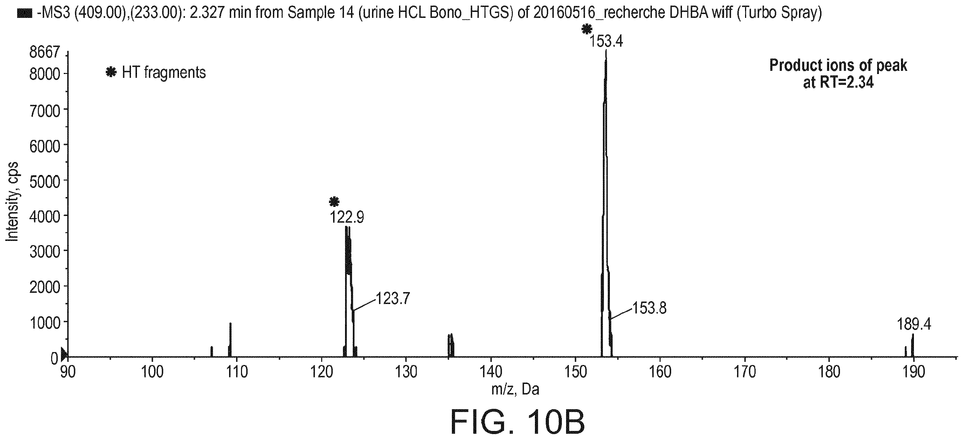

As it had been reported in the literature (Garcia-Villalba et al. Eur J Nutr (2014) 53:1015-1027), the presence of a potential sulfo-glucuronide conjugate of HT ([M-H]- at m/z 409) was investigated. One peak was detected for this mass to charge ratio and the identity was confirmed by MS³ as sulfo-glucuronide conjugate of HT (**FIGS. 10A** and **10B**). Indeed, searching product ions of the first precursor ion at *m*/*z* 409 and second precursor ion at *m*/*z* 233 gave product ions with *m*/*z* at 123 and 153, typical of hydroxytyrosol.

Hydroxytyrosol was also detected in basal samples meaning before the consumption of the olive leaf extract. Hydroxytyrosol, also named 3,4-dihydroxyphenylethanol (DOPET), is a metabolite of the neuro-transmitter dopamine and is already present in the body at small amounts.

The detection and identification of homovanillyl alcohol (HVOH) and its glucuronide and sulfate conjugates (HVOH-G and HVOH-S) was challenging as several peaks were detected when searching them by Multiple Reaction Monitoring (MRM): 343 → 113 for HVOH-G, 247 ---> 167 for HVOH-S and 167 -> 152 for HVOH. This meant that other molecules excreted in urine had a similar pattern of fragmentation to HVOH.

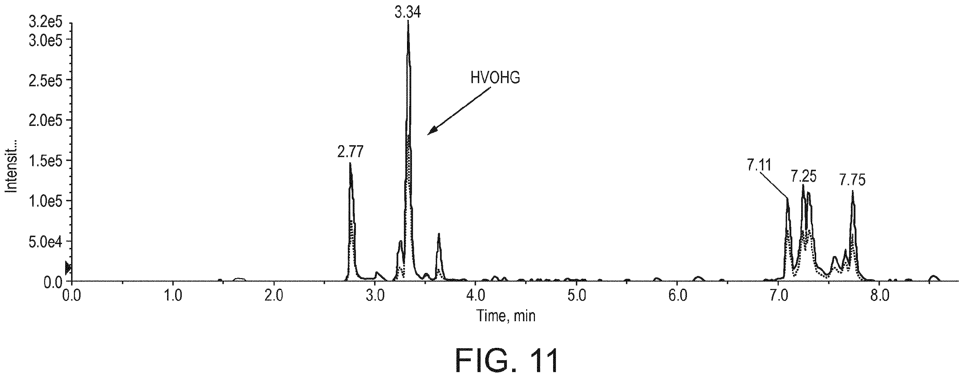

Searching for HVOH-G, only one of the peaks detected by MRM with the transitions 343 → 113 (at RT=3.34 min, **FIG. 11**) could be clearly identified as HVOH glucuronide by comparison with the corresponding chemical standard commercially available. Regarding the peaks obtained for the transition 247 → 167, even by MS³ clear identification of HVOH-S was not possible.

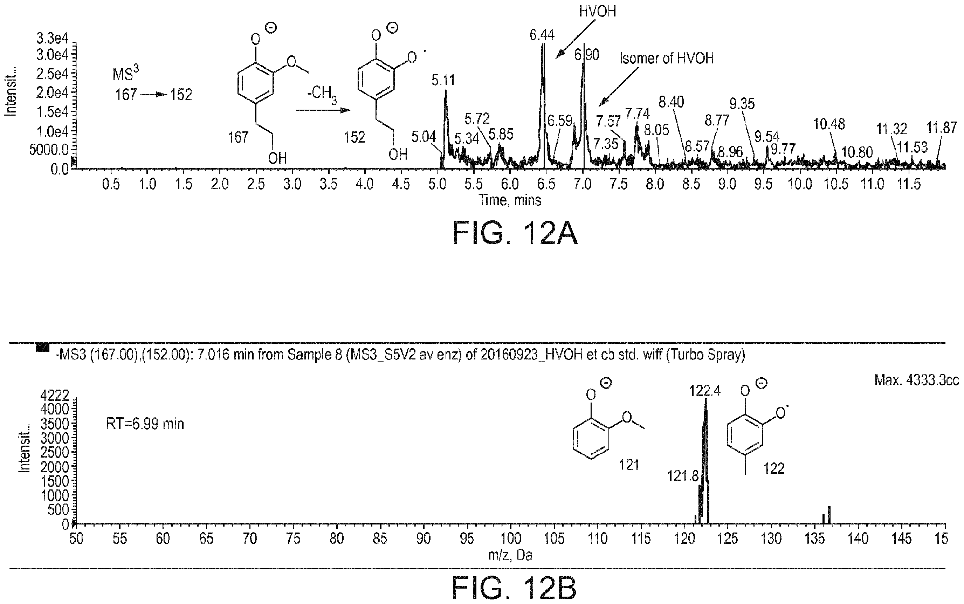

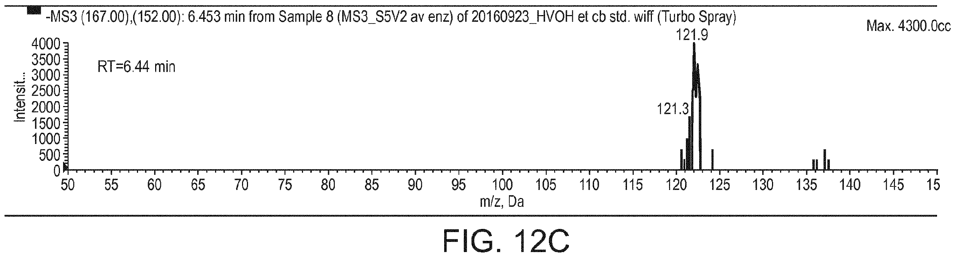

As mentioned above, several peaks were detected by MRM with the transition 167 → 152. MS³ fragmentation confirmed that two peaks had the same CID product ion spectrum as the chemical standard HVOH (**FIGS. 12A-12C**), exhibiting fragment ions at *m*/*z* 121 and 122. The peak at RT=6.44 min is HVOH with the methyl on 3-OH (by comparison with the chemical standard). The peak at RT=6.99 min is tentatively identified as an isomer of HVOH, which could display the methyl group on the 4-OH (**FIGS. 13A** and **B**). This would need to be confirmed by the use of a chemical standard which is not commercially available.

Such as HT, HVOH was detected in samples after intake of the olive leaf extract, as well as in basal samples. HVOH can be a product of methylation of endogenous hydroxytyrosol or of hydroxytyrosol produced in response to alcohol consumption, explaining its presence in basal samples. Theoretically, the methylation of the catechol moiety (on 3-OH or 4-OH of the phenyl ring) can be performed by the catechol-O-methyl-transferase (COMT) on either hydroxyl group of hydroxytyrosol. Thus both forms can be present in the body. However, COMT may favor one hydroxyl over the other (Thakker et al. The Journal of Biological Chemistry Vol. 261, No. 12, Issue of April 25, pp 54044413 1986), suggesting that one of the isomers may be more abundant than the other one.

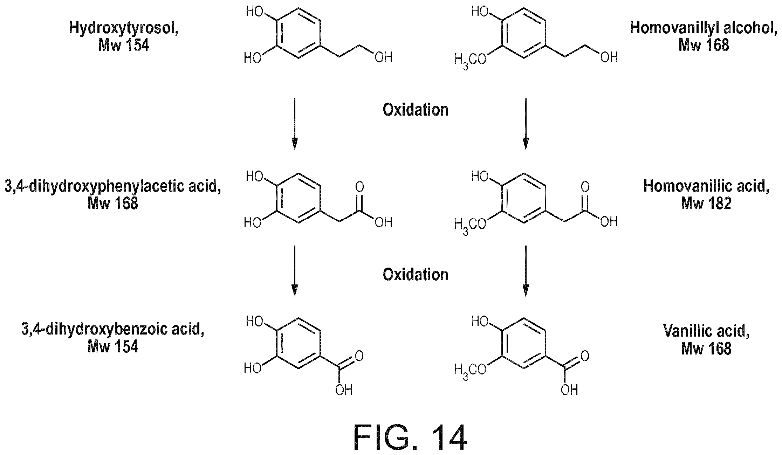

Other phenolic compounds have been described as metabolites of hydroxytyrosol and could also potentially be metabolites of OE. Indeed, 3,4-dihydroxyphenylacetic (DHPA) and homovanillic (HVA) acids have been detected in urine of subjects who had ingested HT solubilized in saline solution and in urine of rats after oral or intravenous administration of HT. DHPA and HVA result from oxidation respectively of HT and HVOH (**FIG. 14**), oxidation still not clarified if due to mammalian and/or microbial enzymatic reaction. Even though not reported so far as a metabolite of HT, 3,4-dihydroxybenzoic acid (3,4-DHBA), also named protocatechuic acid, could be a metabolite of DHPA by oxidation, so an indirect metabolite of HT. Vanillic acid (VA) could be an oxidation product of HVA (**FIG. 14**), so an indirect metabolite of HT.

DHPA, HVA, 3,4-DHBA and VA are commercially available. To facilitate their detection, urine samples were enzymatically treated to release the unconjugated forms of these phenolic compounds. They were all detected in samples after intake of the olive leaf extract and in basal samples, however an evaluation of their quantities in samples was necessary to clarify if their presence was resulting from the ingredient ingestion or from another food source.

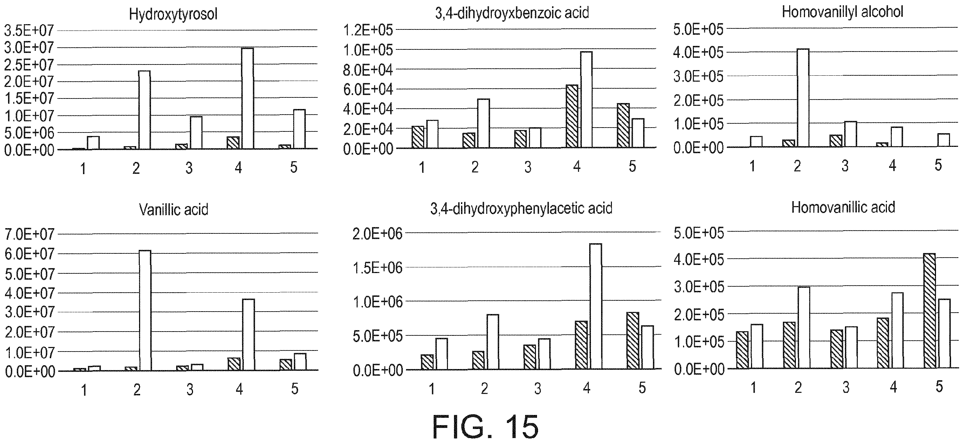

Relative amount of the metabolites identified was first evaluated to clarify if their excretion in urine was increasing after intake of the olive leaf extract. This evaluation was based on the measurement of the area under the corresponding chromatographic peak, which reflects the amount. **FIG. 15** shows that 3,4-dihydroxybenzoic, 3,4-dihydroxyphenylacetic and homovanillic acids do not increase in urine samples of all the subjects after intake of the olive leaf extract. The quantity of vanillic acid strongly increases in urine of subjects 2 and 4, but not as much in subjects 1, 3 and 5. Vanillic acid has been detected in olive leaves (Wang et al. 2013) and is widely used as a flavoring agent so may not be specific to intake of olive leaf extract. Hydroxytyrosol and homovanillyl alcohol are the two metabolites which, even though present in basal samples of some subjects, specifically increase after intake of olive leaf extract. HT and HVOH seem to be the best metabolites to follow the intake of OE from olive leaf extract. The tentatively identified isomer of HVOH also seems to respond to intake of olive leaf extract (not shown).

To have a preliminary estimation of HT and HVOH amounts in urine samples and better target the range of concentration during the method validation, HT and HVOH were quantified using an external calibration curve (standard curve in solvent).

**Table 3 is showing hydroxytyrosol concentration (µmol/L) in urine before versus after intake of olive leaf extract.**

| | **Basal** | | **After Olive Leaf Extract** | | **Specific From Olive Leaf Extract** | |
|---|---|---|---|---|---|---|
| | **Free** | **Total** | **Free** | **Total** | **Free** | **Total** |
| **S1** | 0.00 | 0.09 | 0.17 | 3.41 | 0.17 | 3.24 |
| **S2** | 0.02 | 0.44 | 0.56 | 23.43 | 0.55 | 22.88 |
| **S3** | 0.05 | 1.03 | 0.28 | 8.99 | 0.23 | 8.76 |
| **S4** | 0.15 | 2.61 | 1.53 | 30.05 | 1.38 | 28.67 |
| **S5** | 0.04 | 0.95 | 0.21 | 11.54 | 0.17 | 11.37 |

**Table 4 is a showing homovanillyl alcohol concentration (µmol/L) in urine before versus after intake of olive leaf extract in Example 1 disclosed herein.**

| | **Basal** | | **After Olive Leaf Extract** | | **Specific From Olive Leaf Extract** | |
|---|---|---|---|---|---|---|
| | **Free** | **Total** | **Free** | **Total** | **Free** | **Total** |
| **S1** | 0.00 | 0.00 | 0.24 | 2.43 | 0.24 | 2.18 |
| **S2** | 0.00 | 0.00 | 1.30 | 2.63 | 1.30 | 1.33 |
| **S3** | 0.00 | 0.00 | 0.99 | 1.84 | 0.99 | 0.85 |
| **S4** | 0.10 | 0.17 | 1.11 | 3.49 | 1.11 | 2.38 |
| **S5** | 0.00 | 0.26 | 0.87 | 2.06 | 0.87 | 1.19 |

**Table 5 is showing homovanillyl alcohol isomer concentration (nmol/L) in urine after intake using the chemical standard of HVOH (<4, peak detected bu concentration lower than the last point of the standard curve at 4 nmol/L)**

| | **Free** | **Total** |
|---|---|---|
| **S1** | 70.00 | 295.91 |
| **S2** | <4 | 1767.09 |
| **S3** | <4 | 1222.97 |
| **S4** | <4 | 1476.74 |
| **S5** | <4 | 1482.28 |

**Table 6 is showing estimated amount of oleuropein aglycone (area under the chromatographic peak) in urine before versus after intake of olive leaf extract in Example 1 disclosed herein.**

| | **Basal** | | **After Olive Leaf Extract** | |
|---|---|---|---|---|
| | **Free** | **Total** | **Free** | **Total** |
| **S1** | ND | NO | 1.67E+04 | 4.25E+05 |
| **S2** | NO | NO | 7.66E+04 | 3.63E+06 |
| **S3** | 1.86E+03 | 7.41E+03 | 7.09E+04 | 1.55E+06 |
| **S4** | 1.65E+03 | 7.27E+03 | 1.01E+05 | 2.52E+06 |
| **S5** | 5.44E+03 | 6.75E+03 | 2.02E+04 | 2.03E+06 |

Results show that HT excreted in urine (**Table 3**) is more abundant than HVOH (**Table 4**). HT and HVOH resulting from the intake of the olive leaf extract and the metabolism of OE are excreted in urine mainly as conjugated form. However, free form of both compounds also results from intake of the olive leaf extract.

The concentration of the tentatively identified isomer of HVOH was estimated using the chemical standard of HVOH. Contrary to HVOH, the isomer of HVOH is absent from the basal samples and specific to intake of the olive leaf extract.

Even though a chemical standard is not commercially available to allow an absolute quantification of OEA, the measurement of the area under the chromatographic peak still allows comparison of the samples. The average of the area under the chromatographic peak of OEA (2 × 10⁶) is in the range of the one obtained for HT (7.7 × 10⁶). This strongly suggests that the amount of OEA is not negligible and that its urinary secretion should be followed.

### Conclusion

**Table 7 is summarizing the findings from Example 1 regarding the presence of oleuropein metabolites in urine after intake of olive leaf extract.**

| Compound | | Number of forms confirmed |
|---|---|---|
| Oleuropein (parent compound) | Not detected | |
| Methylated oleuropein aglycone | Not detected | |
| Oleuropein aglycone glucuronide | Presence & identity confirmed | 2 |
| Oleuropein aglycone sulfate | Presence & identity confirmed | 1 |
| Oleuropein aglycone | Presence & identity confirmed | 1 |
| Elenolic aglycone glucuronide | Not detected | |
| Oleuropein aglycone sulfate | Not detected | |
| Oleuropein aglycone | Not detected | |
| Hydroxytyrosol glucuronide | Presence & identity confirmed | 2 |
| Hydroxytyrosol sulfate | Presence & identity confirmed | 1 |
| Hydroxytyrosol sulfoglucuronide | Presence & identity confirmed | 1 |
| Hydroxytyrosol | Presence & identity confirmed | 1 |
| Homovanillyl alcohol glucuronide | Presence & identity confirmed | 1 |
| Homovanillyl alcohol sulfate | Potential presence but identity not fully confirmed | |
| Homovanillyl alcohol | Presence & identity confirmed | 2 |

**Table 7** summarizes the findings. Among the different compounds detected and identified, three are proposed to be representative of intake of olive leaf extract: hydroxytyrosol, homovanillyl alcohol, and oleuropein aglycone. An isomer of homovanillyl alcohol was also identified. This pilot study also improved the chromatographic conditions which had been preliminarily developed using commercial urine samples spiked with chemical standards of the aglycones.

Homovanillyl alcohol has been reported in the literature but not always detected after oleuropein or hydroxytyrosol intake. This must be due to the sensitivity of the methods used, as homovanillyl alcohol is mainly reported when GC-MS was used. To our knowledge, this is the first time that the identified isomer of homovanillyl alcohol is reported.

### Example 2: Chondrocyte Data

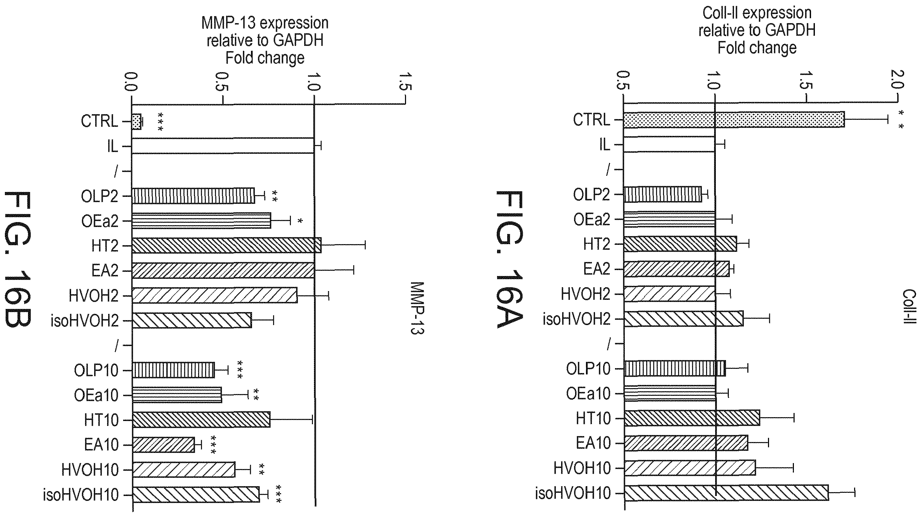

Chondrocytes in alginate beads were subjected to a two-hour polyphenols pretreatment followed by a twenty-four hour IL1-α stimulation. The results are shown in **FIGS.16A-16C****,** which demonstrate that oleuropein metabolites protect against inflammatory-induced catabolism.

Regarding **FIG. 16A****,** Collagen-II is an anabolism-related gene for major structural components of the extracellular matrix of joints. The data regarding this gene showed a trend for a protective effect of polyphenols at 10 µM. The isomer of homovanillyl alcohol, namely 3-hydroxy-4-methoxyphenethanol (or alternatively named 3-hydroxy-4-methoxyphenethyl alcohol) had a significant effect ("isoHVOH").

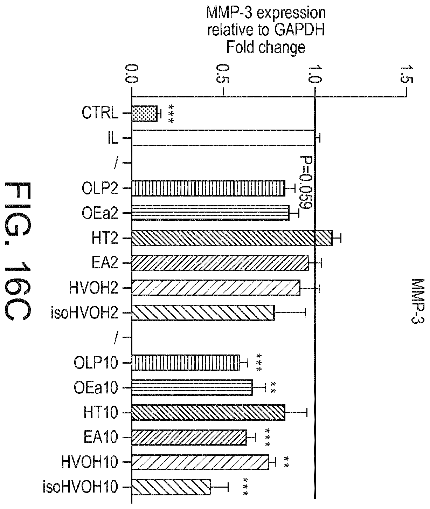

Regarding **FIGS. 16B** and **16C****,** matrix metalloproteinases (e.g., MMP-3 and MMP-13) are implicated in collagen degradation and joint inflammation. Protective effects were found from every tested polyphenol at 10 µM except hydroxytyrosol.

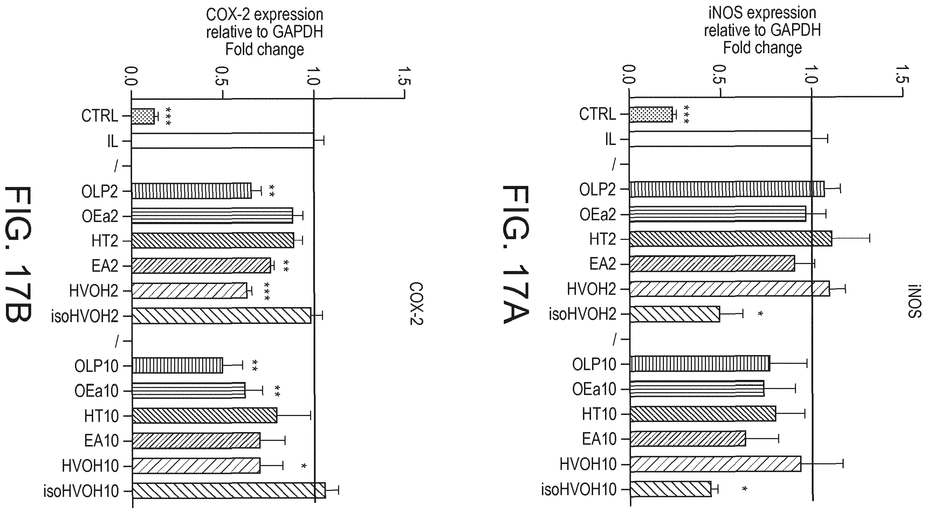

Then the anti-inflammatory effect of oleuropein metabolites in chondrocytes was studied. The results are shown in **FIGS. 17A** and **17B****.**

Regarding **FIG. 17A****,** the inducible isoform of nitric oxide synthase (iNOS) is an inflammation-related gene linked to NO production. The data regarding this gene showed a trend for a protective effect of polyphenols at 10 µM. The isomer of homovanillyl alcohol, namely 3-hydroxy-4-methoxyphenethanol (or alternatively named 3-hydroxy-4-methoxyphenethyl alcohol) had a significant effect at both 2 µM and 10 µM ("isoHVOH").

Regarding **FIG. 17B****,** cyclooxygenase-2 (COX-2) is another inflammation-related gene. It is linked to the Prostaglandin E2 (PGE2) pathway. OLP, EA and HVOH were efficient at 2 µM. OLP, OEA and HVOH were efficient at 10 µM.

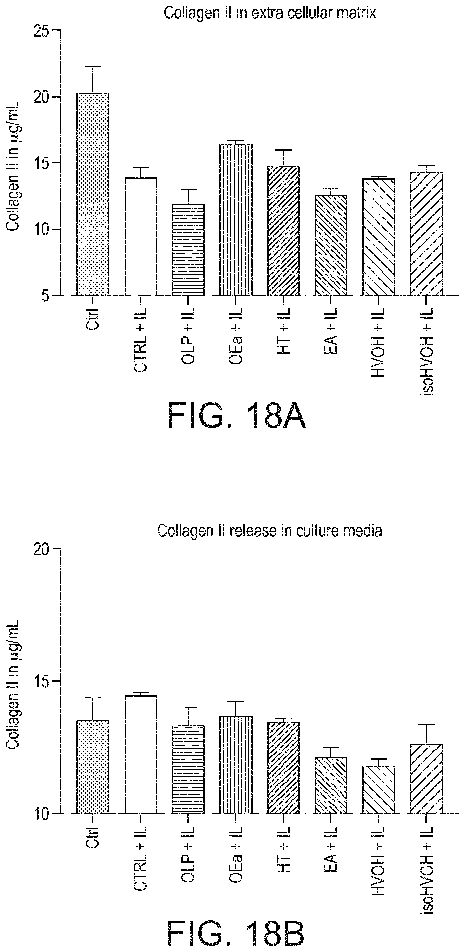

Next it was shown that oleuropein metabolites prevent collagen breakdown in inflamed chondrocytes (**FIGS. 18A and 18B**). In this regard, collagen in extra-cellular matrix corresponds to the collagen synthesized by the cells, i.e., anabolism. **FIG. 18A** demonstrates a protective effect of OEA and HT. Collagen in culture media corresponds to the collagen degraded by cells and released in the media, i.e., catabolism. **FIG. 18B** demonstrates a potent anti-breakdown effect (improved over control) by EA, HVOH and isoHVOH.

## Claims

1. Homovanillyl alcohol and/or 3-hydroxy-4-methoxyphenethanol for use in a method of treating or preventing cartilage breakdown, or reducing incidence of cartilage breakdown, the method comprising administering to an individual in need thereof or at risk thereof a therapeutically or prophylactically effective amount of the homovanillyl alcohol and/or 3-hydroxy-4-methoxyphenethanol .

2. A combination of a precursor of homovanillyl alcohol and/or a precursor of 3-hydroxy-4-methoxyphenethanol and a probiotic capable of converting at least a portion of the precursor or precursors into homovanillyl alcohol and/or 3-hydroxy-4-methoxyphenethanol, for use in a method of treating or preventing cartilage breakdown, or reducing incidence of cartilage breakdown, the method comprising administering the combination to an individual in need thereof or at risk thereof in an amount that provides a therapeutically or prophylactically effective amount of homovanillyl alcohol and/or 3-hydroxy-4-methoxyphenethanol.

3. The composition for use according to Claim 1 or Claim 2 wherein the administration to the individual is daily for at least one month.

4. The composition for use according to Claim 2 wherein the precursor is selected from the group consisting of hydroxytyrosol, oleuropein aglycone, oleuropein, demethyloleuropein, oleuroside, verbascoside and mixtures thereof.

## Patentansprüche

1. Homovanillylalkohol und/oder 3-Hydroxy-4-methoxyphenethanol zur Verwendung in einem Verfahren zum Behandeln oder Vorbeugen von Knorpelschäden oder Verringern eines Vorkommens von Knorpelschäden, das Verfahren umfassend ein Verabreichen einer therapeutisch oder prophylaktisch wirksamen Menge des Homovanillylalkohols und/oder von 3-Hydroxy-4-methoxyphenethanol an ein Individuum, das dies benötigt oder bei dem ein Risiko dafür besteht.

2. Kombination aus einem Vorläufer von Homovanillylalkohol und/oder einem Vorläufer von 3-Hydroxy-4-methoxyphenethanol und einem Probiotikum, das in der Lage ist, mindestens einen Teil des Vorläufers oder der Vorläufer in Homovanillylalkohol und/oder 3-Hydroxy-4-methoxyphenethanol umzuwandeln, zur Verwendung in einem Verfahren zum Behandeln oder Vorbeugen von Knorpelschäden oder Verringern des Vorkommens von Knorpelschäden, das Verfahren umfassend das Verabreichen der Kombination an ein Individuum, das dies benötigt oder bei dem ein Risiko dafür besteht, in einer Menge, die eine therapeutisch oder prophylaktisch wirksame Menge von Homovanillylalkohol und/oder 3-Hydroxy-4-methoxyphenethanol bereitstellt.

3. Zusammensetzung zur Verwendung nach Anspruch 1 oder 2, wobei die Verabreichung an das Individuum täglich für mindestens einen Monat erfolgt.

4. Zusammensetzung zur Verwendung nach Anspruch 2, wobei der Vorläufer aus der Gruppe ausgewählt ist, bestehend aus Hydroxytyrosol, Oleuropein-Aglykon, Oleuropein, Demethyloleuropein, Oleurosid, Verbascosid und Mischungen davon.

## Revendications

1. Alcool homovanillylique et/ou 3-hydroxy-4-méthoxyphénéthanol destinés à être utilisés dans un procédé de traitement ou de prévention de la dégradation du cartilage, ou la réduction de l'incidence de la dégradation du cartilage, le procédé comprenant l'administration à un individu en ayant besoin ou étant à risque d'une quantité thérapeutiquement ou prophylactiquement efficace de l'alcool homovanillylique et/ou de 3-hydroxy-4-méthoxyphénéthanol.

2. Combinaison d'un précurseur de l'alcool homovanillylique et/ou d'un précurseur de 3- hydroxy-4-méthoxyphénéthanol et d'un probiotique capable de convertir au moins une partie du ou des précurseurs en alcool homovanillylique et/ou 3-hydroxy-4-méthoxyphénéthanol, destinée à être utilisée dans un procédé de traitement ou de prévention de la dégradation du cartilage, ou de réduction de l'incidence de la dégradation du cartilage, le procédé comprenant l'administration de la combinaison à un individu en ayant besoin ou étant à risque en une quantité qui fournit une quantité thérapeutiquement ou prophylactiquement efficace de l'alcool homovanillylique et/ou de 3-hydroxy-4-méthoxyphénéthanol.

3. Composition destinée à être utilisée selon la revendication 1 ou la revendication 2, dans laquelle l'administration à l'individu est quotidienne pendant au moins un mois.

4. Composition destinée à être utilisée selon la revendication 2, dans laquelle le précurseur est choisi dans le groupe constitué d'hydroxytyrosol, oleuropéine aglycone, oleuropéine, déméthyloleuropéine, oleurocide, verbascoside et leurs mélanges.
